# EUROPÄISCHE PATENTSCHRIFT

(11) **EP 2 568 035 B1**
(45) Veröffentlichungstag und Bekanntmachung des Hinweises auf die Patenterteilung: **23.07.2014**
(21) Anmeldenummer: 12181111.1
(22) Anmeldetag: 21.08.2012
(51) Int. Cl.: C11B 9/00, C11D 3/50

(54) **Riechstoffmischungen enthaltend bestimmte Isolongifolenylmethylether**
Perfume mixtures containing certain isolongifolenyl methyl ethers
Melanges de parfums comprenant certains ethers de methyle d'isolongifolenyle

(30) Priorität: 09.09.2011 DE 102011082464
(43) Veröffentlichungstag der Anmeldung: 13.03.2013
(73) Patentinhaber: Symrise AG, 37603 Holzminden (DE)
(72) Erfinder: Eh, Marcus, Dr., 37603 Holzminden (DE); Kurzenne, Pierre, 92270 Bois Colombes (FR); vom Ende, Marc, 37619 Bodenwerder (DE); Coppermann, Emilie, 75006 Paris (FR)
(74) Vertreter: Fabry, Bernd

(56) Entgegenhaltungen:
- EP-A1- 1 552 814
- EP-A1- 2 184 339
- EP-A2- 1 178 105
- EP-A2- 1 220 850

## Beschreibung

Die vorliegende Erfindung betrifft primär Riechstoffmischungen, vorzugsweise Parfümöle, umfassend
(a) Verbindung (**A-1**) und Verbindung (**A-2**) 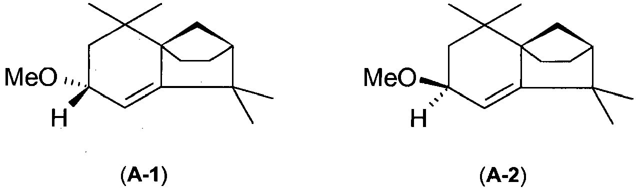 und
(b) einen oder mehrere weitere Moschusriechstoffe, welche keine Isolongifolenylmethylether sind,
wobei vorzugsweise das Massenverhältnis der Gesamtmenge an Moschusriechstoffen des Bestandteils (b) zu der Gesamtmenge der Verbindungen (**A-1**) und (**A-2**) (Bestandteil (a)) größer oder gleich 1 : 2 ist.

Ferner betrifft die vorliegende Erfindung parfümierte Produkte enthaltend solche erfindungsgemäßen Riechstoffmischungen, insbesondere Parfümöle, sowie ein Verfahren zur Modifizierung bestimmter geruchlicher Eigenschaften von Riechstoffen, Riechstoffmischungen, Parfümöle bzw. von entsprechend parfümierten Produkten.

In EP 1 178 105 sind diverse Isolongifolenylalkylether und deren geruchliche Eigenschaften beschrieben. EP 1 178 105 offenbart auch Isolongifolenylmethylether, zu denen auch die beiden isomeren Verbindungen (**A-1**) und (**A-2**) zählen. Als Geruchsbewertung für das Stereoisomer (**A-1**) wird dort Ambra, trocken-Moschus, leicht würzig und schwach holzig-Patchouli angegeben. Stereoisomer (**A-2**) wird dort als Riechstoff charakterisiert mit einem Geruch von Moschus, leicht blumig, Cashmeran, exaltierend und würzig. EP 1 178 105 offenbart ferner für eine Mischung umfassend 37,6 GC-% (**A-1**) und 59,1 GC-% (**A-2**) einen Geruch von stark Moschus, pudrig, Cashmeran, Ambra, exaltierend und schwach holzig.

Mischungen bzw. Kombinationen dieser Isolongifolenylalkylether mit weiteren Moschusriechstoffen, die keine Isolongifolenylalkylether sind, werden in EP 1 178 105 nicht offenbart. EP 1 178 105 offenbart insbesondere keine Mengenverhältnisse und keine geruchlichen Effekte bei Kombination dieser Isolongifolenylalkylether mit davon strukturell verschiedenen Moschusriechstoffen.

Es besteht immer wieder Bedarf in der Parfümerie, bestimmte geruchliche Aspekte eines Riechstoffes oder einer Riechstoffmischung zu modifizieren, zu betonen (d.h. zu verstärken oder hervorzuheben) oder zu maskieren (d.h. zu vermindern oder zu unterdrücken).

Parfümöle und parfümierte Produkte enthaltend einen oder mehrere Moschusriechstoffe sind dem Fachmann auf dem Gebiet der Parfümerie bekannt, siehe beispielsweise EP 1 552 814.

Moschusriechstoffe sind dem Fachmann bekannt und spielen in der Parfümerie eine sehr wichtige Rolle. Dem Fachmann auf dem Gebiet der Parfümerie ist bekannt, dass Moschusriechstoffe, insbesondere makrocyclische Moschusriechstoffe, neben ihren typischen erwünschten Hauptnoten, die einen erogenen, maskulinen und animalischen Geruch bewirken, unerwünschte Nebennoten auftreten können. Solche unerwünschten und störenden Nebennoten, auch Fehlnoten oder Off-Noten genannt, sind dabei metallische Noten, insbesondere Noten nach heissem Bügeleisen (nachfolgend als Bügeleisennote bezeichnet), wie beispielsweise auch für Moschusriechstoffe in EP 1 487 816 und EP 1 220 850 beschrieben.

Ein weiterer nachteiliger Effekt, der im Zusammenhang mit Moschusriechstoffen in einer Riechstoffkomposition oder einem Parfümöl beobachtet werden kann, ist die Absenkung oder Verminderung der Geruchsintensität (d.h. der Gesamtgeruchsstärke) einer solchen Riechstoffkomposition oder eines solchen Parfümöls. Daher besteht regelmäßig auch die Notwendigkeit, der Absenkung oder Verminderung der Geruchsintensität einer solchen Riechstoffkomposition oder eines solchen Parfümöls durch Zugabe von geruchsverstärkenden Substanzen (sogenannter "Enhancer" oder "Booster") entgegenzuwirken.

Die primäre Aufgabe der vorliegenden Erfindung bestand darin, eine Substanz (Stoff oder Stoffgemisch) zu finden, die unerwünschte geruchliche Noten von Moschusriechstoffen, insbesondere von makrocyclische Moschusriechstoffen, zu modifizieren, vorzugsweise zu maskieren, d.h. zu vermindern oder zu unterdrücken vermag. Bei diesen unerwünschten Noten handelt es sich insbesondere um metallische Noten, insbesondere um Geruchsnoten nach heissem Bügeleisen.

Zusätzlich zu der modifizierenden bzw. maskierenden Wirkung sollte die gesuchte Substanz vorzugsweise auch der im Zusammenhang mit Moschusriechstoffen in einer Riechstoffkomposition oder einem Parfümöl regelmäßig beobachteten Absenkung oder Verminderung der Geruchsintensität (d.h. der Gesamtgeruchsstärke) einer solchen Riechstoffkomposition oder eines solchen Parfümöls entgegenwirken können. Es sollte daher vorzugsweise diesbezüglich eine geruchliche Verstärkung oder Betonung bewirkt werden können ("Enhancer"- oder "Booster"-Wirkung).

Gelöst wird diese Aufgabe durch eine erfindungsgemäße Riechstoffmischung, vorzugsweise durch ein erfindungsgemäßes Parfümöl, umfassend
(a) Verbindung (**A-1**) und Verbindung (**A-2**) 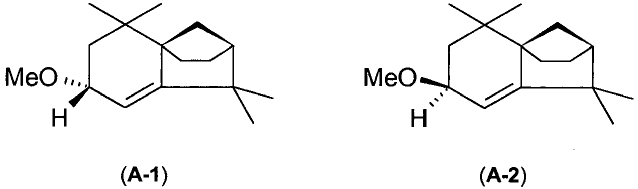 und
(b) einen oder mehrere weitere Moschusriechstoffe, welche keine Isolongifolenylmethylether sind, vorzugsweise einen oder mehrere weitere Moschusriechstoffe, welche keine Isolongifolenyl-C₁-C₄-alkylether sind,
wobei vorzugsweise das Massenverhältnis der Gesamtmenge an Moschusriechstoffen des Bestandteils (b) zu der Gesamtmenge der Verbindungen (**A-1**) und (**A-2**) (Bestandteil (a)) größer oder gleich 1 : 2 ist, bevorzugt größer oder gleich 1 : 1, weiter bevorzugt gröβer oder gleich 3 : 2.

### Bestandteil (a) einer erfindungsgemäßen Riechstoffmischung:

Bestandteil (a) einer erfindungsgemäßen Riechstoffmischung ist eine Diastereomerengemisch. Bestandteil (a) besteht aus den beiden epimeren Verbindungen (**A-1**) und (**A-2**).

Die Verbindungen (**A-1**) und (**A-2**) der oben angegebenen Strukturformeln sind bestimmte Isomere des Isolongifolenylmethylethers und an sich literaturbekannt. Sie können beispielsweise hergestellt werden wie beschrieben in EP 1 178 105 (siehe oben).

Vorzugsweise ist das Massenverhältnis der Gesamtmenge des Isomers (**A-1**) zu der Gesamtmenge des Isomers (**A-2**) größer oder gleich 11 : 4, bevorzugt größer oder gleich 3 : 1, weiter bevorzugt größer oder gleich 7 : 2.

Bevorzugt liegt das Massenverhältnis der Gesamtmenge des Isomers (**A-1**) zu der Gesamtmenge des Isomers (**A-2**) im Bereich von 3 : 1 bis 12 : 1, weiter bevorzugt im Bereich von 7 : 2 bis 10 : 1, besonders bevorzugt im Bereich von 15 : 4 bis 10 : 1, und am meisten bevorzugt im Bereich von 4 :1 bis 8 : 1.

Die erfindungsgemäß einzusetzenden Isomerenmischungen der Verbindungen (**A1**) und (**A2**) wurden im Rahmen der vorliegenden Erfindung analog zu den in EP 1 178 105 beschriebenen Verfahren hergestellt. Solche Isomerenmischungen wurden mittels fraktionierter Destillation der entsprechenden Reaktionsproduktmischung hergestellt, wobei je nach Destillationsbedingungen mehrere unterschiedliche Fraktionen von erfindungsgemäß einzusetzenden Isomerenmischungen erhalten wurden, die erfindungsgemäß als Bestandteil (a) eingesetzt werden können. Alternativ wurden zunächst die Verbindungen (**A1**) und (**A2**) in (weitgehend) reiner Form hergestellt und anschließend das gewünschte Gewichtsverhältnis von (**A1**) zu (**A2**) durch Vermischen der entsprechenden Mengenanteile der beiden reinen Verbindungen (**A1**) und (**A2**) eingestellt.

Die angegebenen bevorzugten Ausgestaltungen für erfindungsgemäße Riechstoffmischungen, insbesondere die als bevorzugt bzw. besonders bevorzugt gekennzeichneten Massenverhältnisse, gelten ebenfalls und entsprechend für erfindungsgemäße Parfümöle, erfindungsgemäße parfümierte Produkte sowie für erfindungsgemäße Verfahren.

Die im Zusammenhang mit der vorliegenden Erfindung beschriebenen modifizierenden Effekte, insbesondere die maskierenden und verstärkenden Effekte, werden am stärksten und deutlichsten beobachtet bei Verwendung der Verbindungen (**A-1**) und (**A-2**) in den als bevorzugt bzw. besonders bevorzugt gekennzeichneten Mengenverhältnissen zueinander, d.h. die bevorzugten angegebenen Isomerenmischungen der Verbindungen (**A-1**) und (**A-2**) des Bestandteils (a).

Dies beschriebenen Effekte treten auch besonders signifikant auf, wenn die genannten Isomerenmischungen der Verbindungen (**A-1**) und (**A-2**) mit weiteren Bestandteilen einer erfindungsgemäßen Riechstoffmischungen, einem erfindungsgemäßen Parfümöl bzw. einem erfindungsgemäßen parfümierten Produkte kombiniert werden, insbesondere in den nachfolgend als bevorzugt bzw. besonders bevorzugt gekennzeichneten Ausgestaltungen.

### Bestandteil (b) einer erfindungsgemäßen Riechstoffmischung_;

Der oder die weiteren Moschusriechstoffe des Bestandteils (b) sind Riechstoffe, die einen Moschusgeruch aufweisen. Solche Riechstoffe sind dem Fachmann bekannt, da "Moschus" (musk) eine sehr wichtige Geruchsrichtung in der Parfümerie darstellt.

Die weiteren Moschusriechstoffe des Bestandteils (b) sind keine Isolongifolenylmethylether, und vorzugsweise keine Isolongifolenyl-C₁-C₄-alkylether.

Als Isolongifolenyl-C₁-C₄-alkylether (entsprechend der nachfolgend links dargestellten Strukturformel) bzw. als Isolongifolenylmethylether (entsprechend der nachfolgend rechts dargestellten Strukturformel) werden im Rahmen der vorliegenden Erfindung sämtliche Verbindungen, einschließlich aller Stereoisomeren, der folgenden Strukturformeln verstanden: 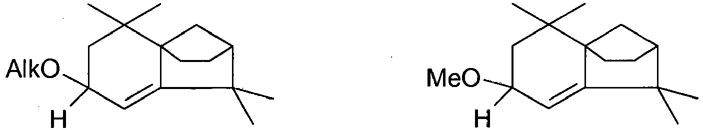 wobei der Rest "Alk" für einen Alkylrest mit 1 bis 4 Kohlenstoffatomen steht und "Me" (wie allgemein üblich) eine Methylgruppe bedeutet.

Die weiteren Moschusriechstoffe des Bestandteils (b) unterscheiden sich somit von Bestandteil (a).

Erfindungsgemäß bevorzugt liegt das Massenverhältnis der Gesamtmenge an weiteren Moschusriechstoffen des Bestandteils (b) zu der Gesamtmenge der Verbindungen des Bestandteils (a) im Bereich von 3 : 2 bis 20 : 1, bevorzugt im Bereich von 3 : 2 bis 15 : 1, besonders bevorzugt im Bereich von 2 : 1 bis 12 : 1.

Erfindungsgemäß bevorzugte Riechstoffmischungen, vorzugsweise Parfümöle, enthalten zwei, drei, vier, fünf oder mehr verschiedene Moschusriechstoffe als Bestandteil (b), wobei jeder der Moschusriechstoffe unabhängig von den übrigen Moschusriechstoffen vorzugsweise gewählt ist aus der Gruppe der makrocyclischen Moschusriechstoffe, der Nitro-Moschusriechstoffe (aromatische Moschusriechstoffe mit Nitrogruppe(n)), der polycyclischen Moschusriechstoffe und/oder der alicyclischen Moschusriechstoffe.

Der oder die weiteren Moschusriechstoffe des Bestandteils (b) sind vorzugsweise gewählt aus der Gruppe der makrocyclischen Moschusriechstoffe, der Nitro-Moschusriechstoffe (aromatische Moschusriechstoffe mit Nitrogruppe(n)), der polycyclischen Moschusriechstoffe und/oder der alicyclischen Moschusriechstoffe, vorzugsweise ein, zwei, drei oder mehr Moschusriechstoffe aus nachfolgender Tabelle 1.

Erfindungsgemäße Riechstoffmischungen enthaltend die Verbindungen (**A-1**) und (**A-2**) sowie einen oder mehrere weitere Moschusriechstoffe des Bestandteils (b), vorzugsweise in denen das Massenverhältnis der Gesamtmenge an weiteren Moschusriechstoffen des Bestandteils (b) zu der Gesamtmenge der Verbindungen (**A-1**) und (**A-2**) (Bestandteils (a)) in den oben (insbesondere den als bevorzugt bzw. besonders bevorzugt) angegebenen Mengenverhältnissen liegt, zeigen eine hinsichtlich der unerwünschten Noten der Moschusriechstoffe des Bestandteils (b) eine modifizierte, insgesamt verbesserte Geruchsqualität.

Im Vergleich zu einer ansonsten identisch zusammengesetzten Vergleichs-Riechstoffmischung, welche die Verbindungen (**A-1**) und (**A-2**) nicht enthält, zeigen die erfindungsgemäßen Riechstoffmischungen weniger ausgeprägte metallische Noten, d.h. die metallischen Noten, insbesondere die Bügeleisennote, der Moschusriechstoffe des Bestandteils (b) werden durch die Anwesenheit der Verbindungen (**A-1**) und (**A-2**) des Bestandteils (a) maskiert, d.h. teilweise oder sogar vollständig vermindert.

Zudem kann durch Zusatz der Verbindungen (**A-1**) und (**A-2**) eine gewisse, meist mäßige bis mittelstark ausgeprägte, Erhöhung der Geruchsintensität (d.h. der Gesamtgeruchsstärke) einer ein oder mehrere Moschusriechstoffe enthaltenden Riechstoffmischung erreicht werden. Die epimeren Verbindungen (**A-1**) und (**A-2**) wirken, insbesondere in den oben angegeben Isomerenverhältnissen, als "Enhancer" oder "Booster" für Moschusriechstoffe und für Riechstoffmischungen und Parfümöle enthaltend ein oder mehrere Moschusriechstoffe.

Bevorzugt werden der oder die Moschusriechstoffe im Rahmen der vorliegenden Erfindung gewählt aus nachfolgender Tabelle 1:

**Tabelle 1:**

| TYP | Produkt / Produktname / Markenname | Name(n) / CAS-Name |
|---|---|---|
| MACRO | EXALTENON | 4-Cyclopentadecen-1-one (4Z)- ; 4-Cyclopentadecen-1-on |
| MACRO | CYCLOHEPTADECENON | 9-Cycloheptadecen-1-on |
| MACRO | CIVETON | 9-Cycloheptadecen-1-on, (9Z)- |
| MACRO | CYCLOHEXADECANOLID, DIHYDROAMBRETTOLID | Oxacycloheptadecan-2-on, ω-Hexadecanolid |
| MACRO | ETHYLENDODECANDIOAT | 1,4-Dioxacyclohexadecane-5,16-dion |
| MACRO | GLOBALIDE® | Oxacyclohexadecen-2-on; 15-Pentadec-(11/12)-enolid |
| MACRO | ETHYLENBRASSYLAT | 1,4-Dioxacycloheptadecane-5,17-dion |
| MACRO | MUSCON | 3-Methy-cyclopentadecanon |
| MACRO | AMBRETTOLID | Oxacycloheptadec-10-en-2-on |
| MACRO | MUSCENON | 3-Methyl-cyclopentadecenon |
| MACRO | VELVIONE®, AMBRETONE | 5-Cyclohexadecen-1-on |
| MACRO | AURELIONE® | 7/8-Cyclohexadecen-1-on |
| MACRO | GLOBANONE® | 8-Cyclohexadecen-1-on |
| MACRO | ISOMUSCONE® | Cyclohexadecanon |
| MACRO | EXALTOLIDE®, MACROLIDE® | 15-Pentadecanolid, Oxacyclohexadecan-2-on, 16-Oxacyclohexadecan-1-on |
| MACRO | COSMONE® | 3-Methyl-(5E/Z)-cyclotetradecen-1-on |
| POLY | TRASEOLIDE® | 1-[2,3-Dihydro-1,1,2,6-tetramethyl-3-(1-methylethyl)-1 H-inden-5- yl]-ethanon |
| POLY | PHANTOLIDE® | 1-(2,3-Dihydro-1,1,2,3,3,6-hexamethyl-1H-inden-5-yl)-ethanon |
| POLY | TONALIDE® | 1-(5,6,7,8-Tetrahydro-3,5,5,6,8,8-hexamethyl-2-naphthalenyl)-ethanon |
| POLY | CRYSOLIDE | 1-[6-(1,1-Dimethylethyl)-2,3-dihydro-1,1-dimethyl-1 H-inden-4-yl]-ethanon |
| POLY | CHROMANOLIDE® | Tetradecansäure, 1-methylethyl ester; Cyclopenta[g]-2-benzopyran, 1,3,4,6,7,8-hexahydro-4,6,6,7,8,8-hexamethyl- |
| POLY | GALAXOLIDE® | Cyclopenta[g]-2-benzopyran, 1,3,4,6,7,8-hexahydro-4,6,6,7,8,8-hexamethyl- |
| ALICYC | HELVETOLIDE® | 1-Propanol,2-[1-(3,3-dimethylcyclohexyl)ethoxy]-2-methyl-, 1-propanoat |
| NITRO | MOSKENE | 2,3-Dihydro-1,1,3,3,5-pentamethyl-4,6-dinitro-1H-Inden |
| NITRO | MUSK TIBETENE | 1-(1,1-Dimethylethyl)-3,4,5-trimethyl-2,6-dinitrobenzol |
| NITRO | ORINOX | 1-[4-(1,1-Dimethylethyl)-2,6-dimethylphenyl]-ethanon |
| NITRO | MUSK XYLOL | 1-(1,1-Dimethylethyl)-3,5-dimethyl-2,4,6-trinitrobenzol |
| NITRO | MUSK KETONE | 1-[4-(1,1-Dimethylethyl)-2,6-dimethyl-3,5-dinitrophenyl]-ethanon |
| NITRO | MUSK ALPHA | 1,3-Dibromo-2-methoxy-4-methyl-5-nitrobenzol |

| | | |
|---|---|---|
| MACRO = makrocyclische Moschusriechstoffe NITRO = Nitro-Mochusriechstoffe POLY = polycyclische Mochusriechstoffe ALICYC = alicyclischer Mochusriechstoff | | |

Weiter bevorzugt als Teil des Bestandteils (b) sind polycyclische und/oder makrocyclische Moschusriechstoffe, wobei sich insbesondere makrocyclische Moschusriechstoffe als besonders vorteilhaft im Sinne der Erfindung gezeigt haben, welche wiederum vorzugsweise gewählt sind aus der Gruppe bestehend aus makrocyclischen C₁₄-C₁₈-Ketonen und makrocyclischen C₁₄-C₁₈-Lactonen, wobei das Keton bzw. Lacton eine Ringgröße von 15 bis 17 Ringatomen und kein, ein oder zwei Sauerstoffatome im Ring aufweist.

Am meisten bevorzugt sind erfindungsgemäße Riechstoffmischungen, deren Bestandteil (b) umfasst oder besteht aus:
(b) 15-Pentadec-(11/12)-enolid, Ethylenbrassylat, 15-Pentadecanolid, 5-Cyclohexadecen-1-on, 7-Cyclohexadecen-1-on, 8-Cyclohexadecen-1-on, 7/8-Cyclohexadecen-1-on, Cyclohexadecanon, 3-Methylcyclopentadecanon, 3-Methylcyclopentadecenon und/oder 9-Cycloheptadecen-1-on, und deren Mischungen.

In einer bevorzugten Ausführungsform wird Bestandteil (b) gewählt aus der Gruppe bestehend aus 15-Pentadec-(11/12)-enolid (Globalide®), Ethylenbrassylat und Oxacyclohexadecan-2-on (Macrolide®) und deren Mischungen.

Entsprechend der vorstehenden Ausführungen betrifft die vorliegende Erfindung ein Verfahren zum Modifizieren, vorzugsweise zum Verringern oder Unterdrücken, einer metallischen Note einer oder mehrerer Moschusverbindungen, vorzugsweise einer oder mehrerer makrocyclischer Moschusverbindungen, umfassend den folgenden Schritt:
- Vermischen einer oder mehrerer Moschusverbindungen, vorzugsweise einer oder mehrerer makrocyclischer Moschusverbindungen, mit einer Gesamtmenge an Verbindungen (**A-1**) und (**A-2**) (Bestandteil (a)) wie oben definiert, die ausreicht, die metallische Note, insbesondere die Bügeleisennote, einer, mehrerer oder sämtlicher der Moschusverbindungen zu modifizieren, vorzugsweise zu verringern oder zu unterdrücken.

### Optionaler Bestandteil (c) einer erfindungsgemäßen Riechstoffmischung:

Vorzugsweise enthält eine erfindungsgemäße Riechstoffmischung, vorzugsweise ein erfindungsgemäßes Parfümöl, zusätzlich zu den Bestandteilen (a) und (b) als einen weiteren Bestandteil
(c) einen, 2, 3, 4, 5 oder mehr Terpenalkohole mit der Summenformel C₁₀HₓO, wobei x die Zahl 18, 20 oder 22 bedeutet, und wobei vorzugsweise ein, mehrere oder sämtliche Terpenalkohole ausgewählt sind aus der Gruppe bestehend aus Linalool, Eucalyptol, Dihydromyrcenol, Citronellol, Nerol, Geraniol, Menthol, Tetrahydrolinalool und Tetrahydrogeraniol.

Bevorzugt enthält eine erfindungsgemäße Riechstoffmischung, vorzugsweise ein erfindungsgemäßes Parfümöl, zusätzlich zu den Bestandteilen (a) und (b) als einen weiteren Bestandteil
(c) einen, 2, 3, 4, 5 oder mehr Terpenalkohole ausgewählt aus der Gruppe bestehend aus Linalool, Eucalyptol, Dihydromyrcenol, Citronellol, Nerol, Geraniol, Menthol, Tetrahydrolinalool und Tetrahydrogeraniol, bevorzugt einen, 2, 3, 4 oder sämtliche Terpenalkohole ausgewählt aus der Gruppe bestehend aus Linalool, Dihydromyrcenol, Citronellol, Nerol und Geraniol.

In einer erfindungsgemäßen Riechstoffmischung, vorzugsweise einem erfindungsgemäßen Parfümöl, ist das Massenverhältnis der Gesamtmenge an Terpenalkoholen (Bestandteil (c)) zu der Gesamtmenge an Verbindungen (**A-1**) und (**A-2**) (Bestandteil (a)) größer oder gleich 1 : 1, bevorzugt größer oder gleich 2 : 1, weiter bevorzugt größer oder gleich 3 : 1, besonders bevorzugt größer oder gleich 4 : 1.

Bevorzugt liegt das Massenverhältnis der Gesamtmenge an Terpenalkoholen (Bestandteil (c)) zu der Gesamtmenge an Verbindungen (**A-1**) und (**A-2**) (Bestandteil (a)) im Bereich von 3 : 1 bis 15 : 1, besonders bevorzugt im Bereich von 4 : 1 bis 10 : 1.

Insbesondere bei Einstellung der vorstehend genannten Massenverhältnisse der Gesamtmenge an Terpenalkoholen (Bestandteil (c)) zu der Gesamtmenge an Verbindungen (**A-1**) und (**A-2**) (Bestandteil (a)) in einer erfindungsgemäßen Riechstoffmischung bzw. einem erfindungsgemäßen Parfümöl, wird die geruchliche Qualität insgesamt verbessert. Durch die Zugabe des Bestandteile (c) werden einer erfindungsgemäßen Riechstoffmischung bzw. einem erfindungsgemäßen Parfümöl nicht nur die dem oder den Terpenalkoholen des Bestandteils (c) eigenen Geruchsnoten hinzugefügt, sondern es entsteht ein darüberhinausgehendes, insgesamt komplexeres Geruchsprofil. Ferner kann eine unerwartete geruchliche Abrundung und eine weitere Verstärkung des Geruchseindrucks beobachtet werden.

Die Terpenalkohole des Bestandteils (c) können natürlichen oder synthetischen Ursprungs sein. Im Falle eines natürlichen Ursprungs können sie Teil eines entsprechenden ätherischen Öls sein oder aus einem solchen angereicht bzw. isoliert worden sein.

### Optionaler Bestandteil (d) einer erfindungsgemäßen Riechstoffmischung:

Vorzugsweise enthält eine erfindungsgemäße Riechstoffmischung, vorzugsweise ein erfindungsgemäßes Parfümöl, zusätzlich zu den Bestandteilen (a) und (b) sowie gegebenenfalls (c) als einen weiteren Bestandteil
(d) einen, 2, 3, oder mehr weitere holzige Riechstoffe (Holzriechstoffe), wobei der weitere holzige Riechstoff bzw. die weiteren holzigen Riechstoffe des Bestandteils (d) von Bestandteil (a) und Bestandteil (b) verschieden ist bzw. sind.

Die Holzriechstoffe des Bestandteils (d) einer erfindungsgemäßen Riechstoffmischung bzw. eines erfindungsgemäßen Parfümöls bewirken in Kombination mit den Bestandteilen (a) und (b) (sowie gegebenenfalls Bestanteil (c)) einer erfindungsgemäßen Riechstoffmischung bzw. eines erfindungsgemäßen Parfümöls einen harmonischeren, natürlicheren, lebendigeren (lebhafteren) und insgesamt wertvolleren Geruchseindruck.

Beispiele für holzige Riechstoffe, die Teil des optionalen Bestandteils (d) sein können, sind dem Fachmann bekannt und beispielsweise zu finden in S. Arctander, Perfume and Flavor Materials, Vol. I und II, Montclair, N. J., 1969, Selbstverlag oder H. Surburg, J. Panten, "Common Fragrance and Flavor Materials", 5th Ed., Wiley-VCH, Weinheim 2006.

Vorzugsweise enthält eine erfindungsgemäße Riechstoffmischung, vorzugsweise ein erfindungsgemäßes Parfümöl eine sensorisch (geruchlich) wirksame Menge des Bestandteils (d), bevorzugt in einer Menge, die ausreicht, einer erfindungsgemäßen Riechstoffmischung, vorzugsweise einem erfindungsgemäßen Parfümöl, eine holzige Note, vorzugsweise eine Sandelholznote, zu verleihen.

Bevorzugte erfindungsgemäße Riechstoffmischungen, vorzugsweise erfindungsgemäße Parfümöle, sind solche, in denen Bestandteil (d) einen, zwei, drei oder mehrere weitere holzige Riechstoffe umfasst oder daraus besteht, ausgewählt aus der Gruppe bestehend aus
(d)(i) 2,3,8,8-Tetramethyl-1,2,3,4,5,6,7,8-octahydro-2-naphthalenylmethylketon und/oder 1-[1,2,3,4,6,7,8,8a-octahydro-1,2,8,8-tetramethylnaphtalen-2-yl]ethanon
   und/oder
(d)(ii) Sandelholzriechstoffen, vorzugsweise ausgewählt aus der Gruppe bestehend aus 2-Methyl-4-(2,2,3-trimethyl-3-cyclopenten-1-yl)butanol (z.B. Brahmanol), 5-(2,2,3-Trimethyl-3-cyclopenten-1-yl)-3-methylpentan-2-ol (z.B. Sandalore®), 2-Ethyl-4-(2,2,3-trimethyl-3-cyclopenten-1-yl)-2-buten-1-ol (z.B. Sandranol®), 3-Methyl-5-(2,2,3-trimethyl-3-cyclopenten-1-yl)-4-penten-2-ol (z.B. Ebanol®), 3,3-Dimethyl-5-(2,2,3-trimethyl-3-cyclopenten-1-yl)-4-penten-2-ol (z.B. Polysantol®), 3-Isocamphylcyclohexanol (z.B. Sandel 80®) und [1-Methyl-2-(1,2,2-trimethyl[3.1.0]hex-3-ylmethyl)cyclopropyl]methanol (z.B. Javanol®).

Erfindungsgemäße Riechstoffmischungen, insbesondere erfindungsgemäße Parfümöle, umfassend Verbindungen (**A-1**) und (**A-2**) (Bestandteil (a)), einen oder mehrere weitere Moschusriechstoffe (Bestandteil (b)) sowie ein, zwei, drei oder mehrere weitere holzige Riechstoffe (Bestandteil (d)) zeigen eine Harmonisierung und Abrundung des Geruchsbildes.

Mischungen enthaltend beide Verbindungen des Bestandteils (d)(i) sind z.B. unter dem Namen Iso E Super@ bekannt und kommerziell erhältlich.

In erfindungsgemäße Riechstoffmischungen, insbesondere erfindungsgemäße Parfümöle, umfassend Verbindungen (**A-1**) und (**A-2**) (Bestandteil (a)), einen oder mehrere weitere Moschusriechstoffe (Bestandteil (b)) sowie Bestandteil (d)(i) 2,3,8,8-Tetramethyl-1,2,3,4,5,6,7,8-octahydro-2-naphthalenylmethylketon und/oder 1-[1,2,3,4,6,7,8,8a-octahydro-1,2,8,8-tetramethylnaphtalen-2-yl]ethanon, insbesondere in den nachfolgend angegebenen Massenverhältnissen, kann ein harmonischerer und komplexerer geruchlicher Effekt beobachtet werden, der die Riechstoffmischung bzw. das Parfümöl insgesamt wertvoller erscheinen lässt.

Eine weitere bevorzugte Ausführungsform betrifft eine erfindungsgemäße Riechstoffmischung, vorzugsweise ein erfindungsgemäßes Parfümöl, wobei das Massenverhältnis der Gesamtmenge an Bestandteil (d)(i) 2,3,8,8-Tetramethyl-1,2,3,4,5,6,7,8-octahydro-2-naphthalenylmethylketon und 1-[1,2,3,4,6,7,8,8a-octahydro-1,2,8,8-tetramethylnaphtalen-2-yl]ethanon zu der Gesamtmenge an Verbindungen (**A-1**) und (**A-2**) (Bestandteil (a)) größer oder gleich 1 : 1 ist, bevorzugt größer oder gleich 2 : 1, weiter bevorzugt größer oder gleich 3 : 1, besonders bevorzugt größer oder gleich 4 : 1.

In einer bevorzugten Ausgestaltung liegt das Massenverhältnis der Gesamtmenge an Bestandteil (d)(i) zu der Gesamtmenge an Verbindungen (**A-1**) und (**A-2**) (Bestandteil (a)) im Bereich von 4 : 1 bis 25 : 1 liegt, weiter bevorzugt im Bereich von 5 : 1 bis 20 : 1, besonders bevorzugt im Bereich von 6 : 1 bis 12 : 1.

Eine weitere bevorzugte Ausführungsform betrifft eine erfindungsgemäße Riechstoffmischung, vorzugsweise ein erfindungsgemäßes Parfümöl, wobei das Massenverhältnis der Gesamtmenge an Sandelholzriechstoffen (Bestandteil (d)(ii)) zu der Gesamtmenge an Verbindungen (**A-1**) und (**A-2**) (Bestandteil (a)) größer oder gleich 1 : 1 ist, vorzugsweise größer oder gleich 2 : 1, bevorzugt größer oder gleich 3 : 1, weiter bevorzugt größer oder gleich 4 : 1.

In einer bevorzugten Ausgestaltung liegt das Massenverhältnis der Gesamtmenge an Bestandteil (d)(ii) zu der Gesamtmenge an Verbindungen (**A-1**) und (**A-2**) (Bestandteil (a)) im Bereich von 2 : 1 bis 20 : 1 liegt, weiter bevorzugt im Bereich von 3 : 1 bis 15 : 1, besonders bevorzugt im Bereich von 4 : 1 bis 10 : 1.

Erfindungsgemäße Riechstoffmischungen, insbesondere erfindungsgemäße Parfümöle, umfassend Verbindungen (**A-1**) und (**A-2**) (Bestandteil (a)), einen oder mehrere weitere Moschusriechstoffe (Bestandteil (b)) sowie Bestandteil (d), insbesondere Bestandteil (d)(ii), zeigen neben einer geruchlichen Harmonisierung und Abrundung zusätzlich cremigere, weichere und/oder milchigere Noten und somit insgesamt einen eleganteren und (noch) wertvolleren Geruchseindruck. Dabei ist insbesondere das Hervorheben der holzigen Weichheit (softness) sowie der cremigen und milchigen Noten der Sandelholzriechstoffe bemerkenswert.

Dies gilt insbesondere für die nachfolgend bevorzugt definierten und insbesondere für die explizit als bevorzugt genannten holzigen Riechstoffe des Bestandteils (d), und insbesondere die Sandelholzriechstoffe des Bestandteils (d)(ii) aus der Gruppe bestehend aus 2-Methyl-4-(2,2,3-trimethyl-3-cyclopenten-1-yl)butanol, 5-(2,2,3-Trimethyl-3-cyclopenten-1-yl)-3-methylpentan-2-ol, 2-Ethyl-4-(2,2,3-trimethyl-3-cyclopenten-1-yl)-2-buten-1-ol, 3-Methyl-5-(2,2,3-trimethyl-3-cyclopenten-1-yl)-4-penten-2-ol, 3,3-Dimethyl-5-(2,2,3-trimethyl-3-cyclopenten-1-yl)-4-penten-2-ol, 3-Isocamphylcyclohexanol und [1-Methyl-2-(1,2,2-trimethyl[3.1.0]hex-3-ylmethyl)cyclopropyl]methanol.

Bevorzugte erfindungsgemäße Riechstoffmischungen, vorzugsweise erfindungsgemäße Parfümöle, sind solche, in denen Bestandteil (c) einen, zwei, drei oder mehr Riechstoffe aus der Gruppe enthält bestehend aus (in Klammern sind ggf. übliche Marken- bzw. Produktnamen angegeben):

Bevorzugte weitere Holzriechstoffe des Bestandteils (d) einer erfindungsgemäßen Riechstoffmischung, die nicht den Bestandteilen (d)(i) und (d)(ii) zuzuordnen sind, sind die folgenden:

Cedrylmethylether (Cedramber), Cedrylmethylketon, Cedrylacetat, (4aR,5R,7aS,9R)-Octahydro-2,2,5,8,8,9a-hexamethyl-4H-4a,9-methanoazuleno(5,6-d)-1,3-dioxol) (Ambrocenide@), 1',1',5',5'-Tetramethylhexahydro-spiro[1.3-dioxolan-2.8'(5'H)-2H-2.4a]methanonaphthalin (Ysamber® K), 1-(2,2,6-Trimethylcydohexyl)hexan-3-ol (Timberol®), Cyclododecylmethylether (Palisandin), (Ethoxymethoxy)cyclododecan (Boisambrene forte®), 3a,6,6,9a-Tetramethyldodecahydronaphtho[2,1-b]furan (Ambroxid®) und 2,2,7,7-Tetramethyltricyclo[6.2.1.0^{1,6}]undecan-6-ol (Terranol®).

In einer bevorzugten Ausführungsform enthält eine erfindungsgemäße Riechstoffmischung, vorzugsweise ein erfindungsgemäßes Parfümöl, neben den Bestandteilen (a) und (b) den Bestandteil (c) sowie den Bestandteil (d).

Sofern eine erfindungsgemäße Riechstoffmischung, ein erfindungsgemäßes Parfümöl oder ein erfindungsgemäßes parfümiertes Produkt einen Bestandteil (d) enthält, der gleichzeitig auch die Kriterien des Bestandteils (b) erfüllt, wird dieser, insbesondere für quantitative Betrachtungen, als Teil des Bestandteils (b) angesehen und auch diesem zugeordnet.

Bevorzugt ist eine erfindungsgemäße Riechstoffmischung, ein erfindungsgemäßes Parfümöl oder ein erfindungsgemäßes Produkt, vorzugsweise in einer der vorstehend als bevorzugt angegebenen Ausgestaltungen, für die bzw. für das gilt:
- das Massenverhältnis der Gesamtmenge an weiteren Moschusriechstoffen des Bestandteils (b) zu der Gesamtmenge an Verbindungen (**A-1**) und (**A-2**) (Bestandteil (a)) liegt im Bereich von 3 : 2 bis 20 : 1, bevorzugt im Bereich von 3 : 2 bis 15 : 1, besonders bevorzugt im Bereich von 2 : 1 bis 12 : 1,
   und/oder
- das Massenverhältnis der Gesamtmenge an Linalool, Eucalyptol, Dihydromyrcenol, Citronellol, Nerol, Geraniol, Menthol, Tetrahydrolinalool und Tetrahydrogeraniol ((bevorzugte Terpenalkohole des Bestandteils (c)) zu der Gesamtmenge an Verbindungen (**A-1**) und (**A-2**) (Bestandteil (a)) ist größer oder gleich 2 : 1, bevorzugt größer oder gleich 3 : 1, und liegt weiter bevorzugt im Bereich von 3 : 1 bis 15 : 1, besonders bevorzugt im Bereich von 4 : 1 bis 10 : 1,
   und/oder
- das Massenverhältnis der Gesamtmenge an Bestandteil (d)(i) 2,3,8,8-Tetramethyl-1,2,3,4,5,6,7,8-octahydro-2-naphthalenylmethylketon und 1-[1,2,3,4,6,7,8,8a-octahydro-1,2,8,8-tetramethylnaphtalen-2-yl]ethanon zu der Gesamtmenge an Verbindungen (**A-1**) und (**A-2**) (Bestandteil (a)) ist größer oder gleich 2 : 1, bevorzugt größer oder gleich 3 : 1, und liegt weiter bevorzugt im Bereich von 4 : 1 bis 25 : 1, weiter bevorzugt im Bereich von 5 : 1 bis 20 : 1, besonders bevorzugt im Bereich von 6 : 1 bis 12 : 1,
   und/oder
- die Gesamtmenge an Bestandteil (d)(ii) zu der Gesamtmenge an Verbindungen (**A-1**) und (**A-2**) (Bestandteil (a)) ist größer oder gleich 2 : 1 und liegt vorzugsweise im Bereich von 2 : 1 bis 20 : 1, weiter bevorzugt im Bereich von 3 : 1 bis 15 : 1, besonders bevorzugt im Bereich von 4 : 1 bis 10 : 1,
jeweils bezogen auf die gesamte Riechstoffmischung, das gesamte Parfümöl oder das gesamte parfümierte Produkt.

In einer besonders bevorzugten Ausgestaltung sind zwei, drei oder sämtliche der vorstehend genannten bevorzugten Ausgestaltungen und/oder Massenverhältnisse bezüglich der Bestandteile (b), (c), (d)(i) und (d)(ii) gleichzeitig eingestellt, wobei wiederum insbesondere zumindest die (bevorzugten bzw. besonders bevorzugten) Massenverhältnisse betreffend Bestandteil (b) eingestellt werden.

Es kann also zusammenfassend festgehalten werden, dass Verbindungen (**A-1**) und (**A-2**) (Bestandteil (a)) zusammen mit dem oder den Moschusriechstoffen des Bestandteils (b) in vorteilhafter Weise in Kombination mit anderen Riechstoffen bestimmte geruchliche Aspekte bewirken.

Mit Blick auf das oben Gesagte kann ferner festgehalten werden, dass Verbindungen (**A-1**) und (**A-2**) (Bestandteil (a)) zusammen mit dem oder den Moschusriechstoffen des Bestandteils (b) in vorteilhafter Weise in Kombination mit anderen Riechstoffen bestimmte geruchliche Aspekte dieser Riechstoffe betont (hervorhebt).

Entsprechend der vorstehenden Ausführungen betrifft die vorliegende Erfindung in einem weiteren Aspekt ein Verfahren zum Erhöhen der Geruchsintensität einer Riechstoffmischung umfassend eine oder mehrere Moschusverbindungen, vorzugsweise umfassend eine oder mehrere makrocyclische Moschusverbindungen, mit folgendem Schritt:
- Vermischen einer oder mehrerer Moschusverbindungen, vorzugsweise einer oder mehrerer makrocyclischer Moschusverbindungen, mit einer Gesamtmenge an Verbindungen (**A-1**) und (**A-2**) wie oben definiert, die ausreicht, die Geruchsintensität der Riechstoffmischung zu erhöhen.

Mit Blick auf das oben Gesagte kann zudem festgehalten werden, dass Verbindungen (**A-1**) und (**A-2**) (Bestandteil (a)), insbesondere zusammen mit dem oder den Moschusriechstoffen des Bestandteils (b), in vorteilhafter Weise in Kombination mit anderen Riechstoffen bestimmte geruchliche Aspekte dieser Riechstoffe betont (hervorhebt).

Entsprechend der vorstehenden Ausführungen betrifft die vorliegende Erfindung in einem weiteren Aspekt ein Verfahren zum Betonen (Hervorheben) bzw. Verstärken eines Geruchs mit einer cremigen, weichen und/oder milchigen Note, vorzugsweise eines oder mehrerer Sandelholzriechstoffe, mit folgendem Schritt:
Vermischen von
   einem oder mehreren Riechstoffen, vorzugsweise gewählt aus den oben bevorzugt genannten Verbindungen des Bestandteils (d)(ii), mit einer cremigen, weichen und/oder milchigen Note, insbesondere mit einer Sandelholznote,
   einem oder mehreren Moschusriechstoffen des Bestandteils (b), vorzugsweise gewählt aus den oben als bevorzugt bezeichneten Moschusriechstoffen des Bestandteils (b), und
   einer Gesamtmenge an Verbindungen (**A-1**) und (**A-2**) (Bestandteil (a)), die ausreicht, den Geruchseindruck des bzw. der Riechstoffe, die eine cremige, weiche und/oder milchige Note, insbesondere eine Sandelholznote, verursachen, zu betonen.

Für die erfindungsgemäßen Verfahren gelten die obigen Angaben zu bevorzugten erfindungsgemäßen Riechstoffmischungen, Parfümölen und parfümierten Produkten entsprechend.

Erfindungsgemäße Riechstoffkompositionen (Riechstoffmischungen), vorzugsweise erfindungsgemäße Parfümöle, können in flüssiger Form, unverdünnt oder mit einem Lösungsmittel verdünnt zur Parfümierung oder Aromatisierung eingesetzt werden. Geeignete Lösungsmittel hierfür sind insbesondere Ethanol, Glycerin, 1,2-Propylenglycol, 1,2-Butylenglycol, Dipropylenglycol, Diethylphtalat, Triethylcitrat, Isopropylmyristat und Triacetin.

Stoffe, mit denen erfindungsgemäße Riechstoffmischungen, insbesondere Parfümöle, kombiniert werden können, sind:

Konservierungsmittel, vorzugsweise die in US 2006/0089413 genannten, Abrasiva, Antiakne-Mittel und Mittel zu Sebumreduktion, vorzugsweise die in WO 2008/046791 genannten, Mittel gegen Hautalterung, vorzugsweise die in WO 2005/123101 genannten, antibakterielle Mittel, Anticellulitis-Mittel, Antischuppen-Mittel, vorzugsweise die in WO 2008/046795 genannten, entzündungshemmende Mittel, irritationsverhindernde Mittel, Antürritantien (antiinflammatorische, irritationshemmende und irritationsverhindernde Mittel), vorzugsweise die in WO 2007/042472 und US 2006/0089413 genannten, antimikrobielle Mittel, vorzugsweise die in WO 2005/123101 genannten, Antioxidantien, vorzugsweise die in WO 2005/123101 genannten, Adstringentien, antiseptische Mittel, Antistatika, Binder, Puffer, Trägermaterialien, vorzugsweise die in WO 2005/123101 genannten, Chelatbildnder, vorzugsweise die in WO 2005/123101 genannten, Zellstimulantien, reinigende Mittel, pflegende Mittel, Enthaarungsmittel, oberflächenaktive Substanzen, deodorierende Mittel und Antiperspirantien, vorzugsweise die in WO 2005/123101 genannten, Weichmacher, Emulgatoren, vorzugsweise die in WO 2005/123101 genannten, Enzyme, ätherische Öle, vorzugsweise die in US 2008/0070825 genannten, Insektenrepellentien, vorzugsweise die in WO 2005/123101 genannten, Fasern, Filmbildner, Fixateure, Schaumbildner, Schaumstabilisatoren, Substanzen zum Verhindern des Schäumens, Schaumbooster, Fungizide, gelierende Mittel und gelbildende Mittel, vorzugsweise die in WO 2005/123101 genannten, Haarpflegemittel, Haarverformungsmittel, Haarglättungsmittel, Feuchtigkeitsregulatoren (feuchtigkeitsspendende, anfeuchtende und/oder feuchthaltende Substanzen), vorzugsweise die in WO 2005/123101 genannten, Osmolyte, vorzugsweise die in WO 2005/123101 genannten, kompatible Solute, vorzugsweise die in WO 01/76572 und WO 02/15686 genannten, bleichende Mittel, stärkende Mittel, fleckenentfernende Mittel, optisch aufhellende Mittel, imprägnierende Mittel, schmutzabweisende Mittel, reibungsverringernde Mittel, Gleitmittel, Feuchtigkeitscremes, Salben, Trübungsmittel, plastifizierende Mittel, deckfähige Mittel, Politur, Glanzmittel, Polymere, vorzugsweise die in WO 2008/046676 genannten, Pulver, Proteine und Proteinhydrolysate, vorzugsweise die in WO 2005/123101 und WO 2008/046676 genannten, rückfettende Mittel, abschleifende Mittel, hautberuhigende Mittel, hautreinigende Mittel, hautpflegende Mittel, hautheilende Mittel (skin repair agents), vorzugsweise enthaltend Cholesterin und/oder Fettsäuren und/oder Ceramide und/oder Pseudoceramide, dabei bevorzugt die in WO 2006/053912 genannten, Hautaufhellungsmittel, vorzugsweise die in WO 2007/110415 genannten, hautschützende Mittel, hauterweichende Mittel, hautkühlende Mittel, vorzugsweise die in WO 2005/123101 genannten, hautwärmende Mittel, vorzugsweise die in WO 2005/123101 genannten, Stabilisatoren, UV-absorbierende Mittel und UV-Filter, vorzugsweise die in WO 2005/123101 genannten, Benzyliden-betadicarbonylverbindungen, vorzugsweise die in WO 2005/107692 genannten, alpha-Benzoylzimtsäurenitrile, vorzugsweise die in WO 2006/015954 genannten, AhR-Rezeptor-Antagonisten, vorzugsweise die in WO 2007/128723 und WO 2007/060256 genannten, Waschmittel, Weichspüler, suspendierende Mittel, Hautbräunungsmittel, vorzugsweise die in WO 2006/045760 genannten, Verdickungsmittel, Vitamine, vorzugsweise die in WO 2005/123101 genannten, Öle, Wachse und Fette, vorzugsweise die in WO 2005/123101 genannten, Phospholipide, vorzugsweise die in WO 2005/123101 genannten, Fettsäuren (gesättigte Fettsäuren, ein- oder mehrfach ungesättigte Fettsäuren, α-Hydroxysäuren, Polyhydroxyfettsäuren), vorzugsweise die in WO 2005/123101 genannten, Verflüssiger, Farbstoffe und farbschützende Mittel sowie Pigmente, vorzugsweise die in WO 2005/123101 genannten, Antikorrosiva, Aromen und Geschmackstoffe sowie weitere zusätzliche Riechstoffe, vorzugsweise die in S. Arctander, Perfume and Flavor Chemicals, Eigenverlag, Montclair, N.J., 1969 und Surburg, Panten, Common Fragrance and Flavor Materials, 5th Edition, Wiley-VCH, Weinheim 2006 aufgeführten, insbesondere die in US 2008/0070825 explizit genannten weiteren Riechstoffe, die nicht bereits Teil der Bestandteile (a) und (b) sowie gegebenenfalls (c) und/oder (d) einer erfindungsgemäßen Riechstoffmischung oder eines erfindungsgemäßen Parfümöls sind, Alkohole und Polyole, vorzugsweise die in WO 2005/123101 genannten, Tenside, vorzugsweise die in WO 2005/123101 genannten, tierische Extrakte, Hefeextrakte, Extrakte von Algen oder Mikroalgen, Elektrolyte, Verflüssiger, organische Lösungsmittel, vorzugsweise die in WO 2005/123101 genannten, oder Silikone und Silikonderivate, vorzugsweise die in WO 2008/046676 genannten.

Des weiteren können erfindungsgemäße Riechstoffmischungen, insbesondere Parfümöle, an einem Trägerstoff adsorbiert sein, der sowohl für eine feine Verteilung der darin enthaltenen Riechstoffe im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer, Cellulose-basierende Stoffe, Zucker, Dextrine (z.B. Maltodextrin) oder Kunststoffe wie PVC, Polyvinylacetate oder Polyurethane sein. Die Kombination aus erfindungsgemäßer Komposition und Trägerstoff stellt einen beispielhaften erfindungsgemäßen Artikel dar.

Erfindungsgemäße Riechstoffmischungen, insbesondere Parfümöle können auch mikroverkapselt, sprühgetrocknet, als Einschluss-Komplexe oder als Extrusions-Produkte (d. h. erfindungsgemäße Produkte) vorliegen und in dieser Form z. B. einem zu parfümierenden Produkt hinzugefügt werden.

Gegebenenfalls können die Eigenschaften der derart modifizierten Kompositionen durch sog. "Coaten" mit geeigneten Materialien im Hinblick auf eine gezieltere Duftfreisetzung weiter optimiert werden, wozu vorzugsweise wachsartige Kunststoffe wie z.B. Polyvinylalkohol verwendet werden. Die resultierenden Produkte stellen wiederum erfindungsgemäße Produkte dar.

Die Mikroverkapselung der erfindungsgemäßen Riechstoffmischungen, vorzugsweise der erfindungsgemäßen Parfümöle, kann beispielsweise durch das sogenannte Koazervationsverfahren mit Hilfe von Kapselmaterialien z.B. aus polyurethanartigen Stoffen oder Weichgelatine, erfolgen. Die sprühgetrockneten Riech- oder Aromastoffkompositionen können beispielsweise durch Sprühtrocknung einer die erfindungsgemäße Riechstoffmischung, vorzugsweise ein Parfümöl, enthaltenden Emulsion, bzw. Dispersion hergestellt werden, wobei als Trägerstoffe modifizierte Stärken, Proteine, Dextrin und pflanzliche Gummen verwendet werden können. Einschluss-Komplexe können z.B. durch Eintragen von Dispersionen der erfindungsgemäßen Riechstoffmischung, vorzugsweise einem erfindungsgemäßen Parfümöl, und Cyclodextrinen oder Harnstoffderivaten in ein geeignetes Lösungsmittel, z.B. Wasser, hergestellt werden. Extrusions-Produkte können durch Verschmelzen einer erfindungsgemäßen Riechstoffmischung, vorzugsweise eines erfindungsgemäßen Parfümöls, mit einem geeigneten wachsartigen Stoff und durch Extrusion mit nachfolgender Erstarrung, ggf. in einem geeigneten Lösungsmittel, z.B. Isopropanol, erhalten werden.

Erfindungsgemäße Riechstoffmischungen, vorzugsweise erfindungsgemäße Parfümöle, können vorzugsweise als solche, in konzentrierter Form, in Lösungen oder in oben beschriebener modifizierter Form verwendet werden für die Herstellung von erfindungsgemäßen parfümierten Produkten.

Bevorzugte erfindungsgemäße parfümierte Produkte enthalten eine erfindungsgemäße Riechstoffmischungen, vorzugsweise ein erfindungsgemäßes Parfümöl, in einer sensorisch wirksamen Menge.

Die Gesamtmasse der Verbindungen (**A-1**) und (**A-2**) (Bestandteil (a)) in einer erfindungsgemäßen Riechstoffmischung (wie oben definiert), vorzugsweise in einer der als bevorzugt gekennzeichneten Ausgestaltungen), oder in einem parfümierten Produkt (wie oben definiert), vorzugsweise in einer der als bevorzugt gekennzeichneten Ausgestaltungen), liegt vorzugsweise im Bereich von 0,25 bis 10 Gew.-% liegt, bevorzugt im Bereich von 0,5 bis 8 Gew.-%, besonders bevorzugt im Bereich von 0,75 bis 7,5 Gew.-%, insbesondere bevorzugt im Bereich von 1 bis 6 Gew.-%, jeweils bezogen auf die Gesamtmasse aller in der Riechstoffmischung bzw. dem parfümierten Produkt enthaltenen Riechstoffe.

Dabei bezieht sich die Gesamtmasse aller in der Riechstoffmischung bzw. dem parfümierten Produkt enthaltenen Riechstoffe vorzugsweise auf die Gesamtmasse aller Riechstoffe mit einer Molmasse von kleiner oder gleich 300 g/mol, bevorzugt mit einer Molmasse von kleiner oder gleich 290 g/mol, besonders bevorzugt mit einer Molmasse von kleiner oder gleich 280 g/mol).

Die oben beschriebenen geruchlichen Effekte, insbesondere die diversen modifizierenden und maskierenden Effekte, treten insbesondere in den vorstehend als bevorzugt bzw. besonders bevorzugt angegebenen Mengenanteilen auf. Die modifizierenden Effekte treten in den Hintergrund und werden nicht mehr in dem gewünschten Maße beobachtet, wenn die Gesamtmasse der Verbindungen (**A-1**) und (**A-2**) bezogen auf die Gesamtmasse aller in einer Riechstoffmischung bzw. einem parfümierten Produkt enthaltenen Riechstoffe, zu hoch gewählt ist (d.h. deutlich oberhalb der oben angegebenen Mengenbereiche), da in einem solchen Fall der Eigengeruch der Verbindungen (**A-1**) und (**A-2**) zu stark in den Vordergrund tritt und die gewünschten modifizierenden Effekte mit zunehmender Überschreitung der obigen angegebenen Mengenanteile überdeckt bzw. weniger stark wahrgenommen werden.

Der Anteil einer erfindungsgemäßen Riechstoffmischung, vorzugsweise eines erfindungsgemäßen Parfümöls, in erfindungsgemäßen parfümierten Produkten beträgt typischerweise 0,01 bis 10 Gew.-%, bevorzugt 0,1 bis 5 Gew.-% und besonders bevorzugt 0,25 bis 3 Gew.-%, jeweils bezogen auf die Gesamtmasse des parfümierten Produktes.

Bevorzugte erfindungsgemäße parfümierte Produkte sind Wasch- und Reinigungsmittel, Hygiene- oder Pflegeprodukte, insbesondere im Bereich der Körper- und Haarpflege, der Kosmetik und des Haushalts.

Bevorzugte erfindungsgemäße parfümierte Produkte sind gewählt aus folgender Liste: Parfüm-Extraits, Eau de Parfums, Eau de Toilettes, Rasierwässer, Eau de Colognes, Pre-shave-Produkte, Splash-Colognes und parfümierte Erfrischungstücher, saure, alkalische und neutrale Reinigungsmittel, wie z.B. Fußbodenreiniger, Fensterglasreiniger, Geschirrspülmittel, Bad- und Sanitärreiniger, Scheuermilch, feste und flüssige WC-Reiniger, pulver- und schaumförmige Teppichreiniger, Textilerfrischer, Bügelhilfen, flüssige Waschmittel, pulverförmige Waschmittel, Wäschevorbehandlungsmittel wie Bleichmittel, Einweichmittel und Fleckenentferner, Wäscheweichspüler, Waschseifen, Waschtabletten, Desinfektionsmittel, Oberflächendesinfektionsmittel, Luftverbesserer in flüssiger, gelartiger oder auf einem festen Träger aufgebrachter Form, Aerosolsprays, Wachse und Polituren wie Möbelpolituren, Fußbodenwachse, Schuhcremes, Körperpflegemittel wie z.B. feste und flüssige Seifen, Duschgele, Shampoos, Rasierseifen, Rasierschäume, Badeöle, kosmetische Emulsionen vom Öl-in-Wasser-, vom Wasser-in-Öl- und vom Wasser-in-Öl-in-Wasser-Typ wie z.B. Hautcremes- und -lotionen, Gesichtscremes und -lotionen, Sonnenschutzcremes-und -lotionen, After-sun-cremes und -lotionen, Handcremes und - lotionen, Fußcremes und -lotionen, Enthaarungscremes und -lotionen, After-shave-Cremes und -lotionen, Hautbräunungscremes und -lotionen, Hautaufhellungscremes und -lotionen, Haarpflegeprodukte wie Haarsprays, Haargele, festigende Haarlotionen, Haarspülungen, permanente und semipermanente Haarfärbemittel, Haarverformungsmittel wie Kaltwellen und Haarglättungsmittel, Haarwässer, Haarcremes und -lotionen, Deodorantien und Antiperspirantien wie z.B. Achselsprays, Roll-ons, Deosticks, Deocremes, Produkte der dekorativen Kosmetik wie z.B. Lidschatten, Nagellacke, Makeups, Lippenstifte, Mascara sowie Kerzen, Lampenöle, Räucherstäbchen, Insektizide und Repellentien.

Besonders bevorzugte erfindungsgemäße parfümierte Produkte sind gewählt aus folgender Liste:
- Eau de Parfums, Eau de Toilettes, Rasierwässer (After-shave), Eau de Colognes, Pre-shave-Produkte, Splash-Colognes;
- saure, alkalische und neutrale Reinigungsmittel, insbesondere im Haushaltsbereich, vorzugsweise Fußbodenreiniger, Fensterglasreiniger, Geschirrspülmittel, Bad- und Sanitärreiniger, Scheuermilch, feste und flüssige WC-Reiniger, pulver- und schaumförmige Teppichreiniger, flüssige Waschmittel, pulverförmige Waschmittel, Wäscheweichspüler, Oberflächendesinfektionsmittel, insbesondere für harte Oberflächen (hard surface cleaner);
- Körperpflegemittel, vorzugsweise feste und flüssige Seifen, Duschgele, Shampoos, Rasierseifen, Rasierschäume;
- kosmetische Emulsionen vom Öl-in-Wasser-, vom Wasser-in-Öl- und vom Wasser-in-Öl-in-Wasser-Typ, vorzugsweise Hautcremes- und -lotionen, Gesichtscremes und -lotionen, Sonnenschutzcremes-und -lotionen, After-sun-cremes und -lotionen, Handcremes und -lotionen, Fußcremes und -lotionen, Enthaarungscremes und - lotionen, After-shave-Cremes und -lotionen, Hautbräunungscremes und -lotionen, Hautaufhellungscremes und -lotionen;
- Haarpflegeprodukte, vorzugsweise Haarsprays, Haargele, festigende Haarlotionen, Haarspülungen, permanente und semipermanente Haarfärbemittel, Haarwässer, Haarcremes und -lotionen;
- Deodorantien und Antiperspirantien, vorzugsweise Achselsprays, Roll-ons (vorzugsweise als alkoholische oder nicht-alkoholische Lösung, als Gel oder (Micro)Emulsion), Deosticks (Deo-stifte), Deocremes.

Die nachfolgenden Beispiele erläutern die Erfindung; sofern nicht anders angegeben beziehen sich Anteile und Prozente auf das Gewicht. In den nachfolgenden exemplarischen erfindungsgemäßen Parfümölen und parfümierten Produkten wurden die vorstehend beschriebenen geruchlichen Effekte beobachtet.

### Beispiele

### Verwendete Abkürzungen:

Dipropylenglycol (DPG), Diethylphthalat (DEP), Triethylcitrat (TEC), Isopropylmyristat (IPM); nat. = natürlich; makrocyclischer Moschusriechstoff (Bestandteil (b) der erfindungsgemäßen Riechstoffmischung (MACRO)); polycyclischer Moschusriechstoff (Bestandteil (b) der erfindungsgemäßen Riechstoffmischung (POLY)).alicyclischer Moschusriechstoff (Bestandteil (b) der erfindungsgemäßen Riechstoffmischung (ALICYC));

HEDIONE® HC/30 enthält mindestens 30 Gew.-% des cis-Isomers und knapp 70 Gew.-% des trans-Isomers von Methyldihydrojasmonat.

Für Erläuterungen der Produktnamen der Riechstoffe siehe z.B. S. Arctander, Perfume and Flavor Materials, Vol. I und II, Montclair, N. J., 1969, Selbstverlag oder H. Surburg, J. Panten, "Common Fragrance and Flavor Materials", 5th Ed., Wiley-VCH, Weinheim 2006.

Die nachfolgende eingesetzten Mischungen (**Mix1),** (**Mix2**) und (**Mix3**) der erfindungsgemäß einzusetzenden Verbindungen (**A1**) und (**A2**) wurden analog zu den in EP 1 178 105 beschriebenen Verfahren hergestellt, wobei die Isomerenmischungen entweder mittels Fraktionierung der entsprechenden Reaktionsgemische erhalten wurden oder zunächst die Verbindungen (**A1**) und (**A2**) in möglichst reiner Form hergestellt wurden und anschließend das jeweilige Gewichtsverhältnis von (**A1**) zu (**A2**) durch Vermischen der entsprechenden Mengenanteile der beiden reinen Verbindungen (**A1**) und (**A2**) gezielt und präzise eingestellt wurde.

Die nachfolgend eingesetzte Mischung (**Mix1**) enthielt die Verbindungen (**A1**) und (**A2**) im Gewichtsverhältnis 3,6 : 1. Die Menge der beiden Verbindungen (**A1**) und (**A2**) lag bei 96,1 Gew.-%, bezogen auf das Gesamtgewicht von (**Mix1**).

Die nachfolgend eingesetzte Mischung (**Mix2**) enthielt die Verbindungen (**A1**) und (**A2**) im Gewichtsverhältnis 4,5 : 1. Die Menge der beiden Verbindungen (**A1**) und (**A2**) lag bei 97,5 Gew.-%, bezogen auf das Gesamtgewicht von (**Mix2**).

Die nachfolgend eingesetzte Mischung (**Mix3**) enthielt die Verbindungen (**A1**) und (**A2**) im Gewichtsverhältnis 5,8 : 1. Die Menge der beiden Verbindungen (**A1**) und (**A2**) lag bei 98,2 Gew.-%, bezogen auf das Gesamtgewicht von (**Mix3**).

### Parfümöl-Beispiel P1:

| | **Ref-P1** | **P1** | **P1a** |
|---|---|---|---|
| AMBRETTOLID (MACRO) | 10,00 | 10,00 | 10,00 |
| AMBROXID 10% in IPM | 10,00 | 10,00 | 10,00 |
| BENZYLACETAT | 20,00 | 20,00 | 20,00 |
| BENZYLSALICYLAT | 15,00 | 15,00 | 15,00 |
| BERGAMOTTEÖL, bergaptenfrei | 60,00 | 60,00 | 60,00 |
| CALONE® 1951 10% in DPG | 15,00 | 15,00 | 15,00 |
| COUMARIN | 5,00 | 5,00 | 5,00 |
| CYCLOGALBANAT® 10% in DPG | 10,00 | 10,00 | 10,00 |
| ALPHA -DAMASCON 1 % in DPG | 20,00 | 20,00 | 20,00 |
| DIHYDROMYRCENOL | 10,00 | 10,00 | 10,00 |
| ETHYLLINALOOL | 75,00 | 75,00 | 75,00 |
| ETHYLLINALYLACETAT | 50,00 | 50,00 | 50,00 |
| ETHYLMALTOL 1 % in DEP | 10,00 | 10,00 | 10,00 |
| ETHYLENBRASSYLAT (MACRO) | 90,00 | 90,00 | 90,00 |
| FLOROSA | 40,00 | 40,00 | 40,00 |
| GERANYLACETAT | 10,00 | 10,00 | 10,00 |
| HEDIONE® HC/30 | 35,00 | 35,00 | 35,00 |
| HEDIONE® | 210,00 | 210,00 | 210,00 |
| HELIONAL® | 15,00 | 15,00 | 15,00 |
| HELVETOLIDE® (ALICYC) | 30,00 | 30,00 | 30,00 |
| HEXENYLSALICYLAT CIS-3 | 20,00 | 20,00 | 20,00 |
| ISO E SUPER® | 40,00 | 40,00 | 40,00 |
| LEAFOVERT® 10% in DEP | 10,00 | 10,00 | 10,00 |
| LILIAL® | 80,00 | 80,00 | 80,00 |
| LYRAL® | 20,00 | 20,00 | 20,00 |
| MANDARINENÖL | 10,00 | 10,00 | 10,00 |
| STYRALYLACETAT | 5,00 | 5,00 | 5,00 |
| SYMROSE® | 15,00 | 15,00 | 15,00 |
| VANILLIN 10% in DEP | 20,00 | 20,00 | 20,00 |
| DIPROPYLENGLYCOL (DPG) | 40,00 | 10,00 | 10,00 |
| MISCHUNG (**Mix2**) | - | 30,00 | - |
| MISCHUNG (**Mix1**) | - | - | 30,00 |
| TOTAL: | 1.000,00 | 1.000,00 | 1.000,00 |

### Parfümöl-Beispiel P2:

| | **Ref-P2** | **P2** | **P2a** |
|---|---|---|---|
| AMAROCIT® | 10,00 | 10,00 | 10,00 |
| AMBROCENIDE® 10% in DPG | 5,00 | 5,00 | 5,00 |
| AMBROXID | 15,00 | 15,00 | 15,00 |
| AURELIONE® (7/8-Cyclohexadecenon) (MACRO) | 70,00 | 70,00 | 70,00 |
| BERGAMOTTEÖL, bergaptenfrei | 90,00 | 90,00 | 90,00 |
| CALONE® 1951 10% in DPG | 20,00 | 20,00 | 20,00 |
| KÜMMELÖL (CARAWAY OIL) | 10,00 | 10,00 | 10,00 |
| CITRAL | 20,00 | 20,00 | 20,00 |
| COUMARIN | 10,00 | 10,00 | 10,00 |
| ALPHA-DAMASCON 1 % in DPG | 15,00 | 15,00 | 15,00 |
| DIHYDROMYRCENOL | 70,00 | 70,00 | 70,00 |
| ESTRAGONOL | 10,00 | 10,00 | 10,00 |
| ETHYLLI NALOOL | 100,00 | 100,00 | 100,00 |
| ETHYLLINALYLACETAT | 90,00 | 90,00 | 90,00 |
| EUGENOL | 10,00 | 10,00 | 10,00 |
| EVERNYL® | 5,00 | 5,00 | 5,00 |
| FRUCTATE® | 5,00 | 5,00 | 5,00 |
| GERANIUMÖL | 5,00 | 5,00 | 5,00 |
| HEDIONE® HC/30 | 100,00 | 100,00 | 100,00 |
| HELIONAL® | 10,00 | 10,00 | 10,00 |
| INDOL 10% in DPG | 5,00 | 5,00 | 5,00 |
| ISO E SUPER® | 100,00 | 100,00 | 100,00 |
| KEPHALIS® | 5,00 | 5,00 | 5,00 |
| LAVENDELÖL | 40,00 | 40,00 | 40,00 |
| CITRONENÖL | 80,00 | 80,00 | 80,00 |
| LILIAL® | 30,00 | 30,00 | 30,00 |
| MANDARINENÖL | 20,00 | 20,00 | 20,00 |
| MUSCENON (MACRO) | 5,00 | 5,00 | 5,00 |
| SANDRANOL® | 10,00 | 10,00 | 10,00 |
| VANILLIN 10% in DPG | 5,00 | 5,00 | 5,00 |
| DIPROPYLENGLYCOL | 30,00 | 10,00 | 10,00 |
| MISCHUNG (**Mix2**) | - | 20,00 | - |
| MISCHUNG (**Mix3**) | - | - | 20,00 |
| TOTAL: | 1.000,00 | 1.000,00 | 1.000,00 |

### Parfümöl-Beispiel P3:

| | **Ref-P3** | **P3** | **P3a** |
|---|---|---|---|
| ALDEHYD C10 (n-Decanal) 10% in DPG | 20,00 | 20,00 | 20,00 |
| ALDEHYD C11 (n-Undecanal) 10% in DPG | 5,00 | 5,00 | 5,00 |
| ALDEHYD C12 (n-Dodecanal) 10% in DPG | 15,00 | 15,00 | 15,00 |
| AMBRETTOLID (MACRO) | 5,00 | 5,00 | 5,00 |
| AMBROCENIDE® 1% in DPG | 20,00 | 20,00 | 20,00 |
| AURELIONE® (MACRO) | 30,00 | 30,00 | 30,00 |
| BENZYLACETAT | 30,00 | 30,00 | 30,00 |
| CITRONELLOL | 15,00 | 15,00 | 15,00 |
| ETHYLVANILLIN 1% in DPG | 20,00 | 20,00 | 20,00 |
| ETHYLENBRASSYLAT (MACRO) | 70,00 | 70,00 | 70,00 |
| FRUCTATE® 10% in DPG | 20,00 | 20,00 | 20,00 |
| GERANYLACETAT | 10,00 | 10,00 | 10,00 |
| GLOBALlDE® (MACRO) | 30,00 | 30,00 | 30,00 |
| HEDIONE® | 30,00 | 30,00 | 30,00 |
| ALPHA-HEXYLZIMTALDEHYD | 90,00 | 90,00 | 90,00 |
| INDOL 10% in DPG | 5,00 | 5,00 | 5,00 |
| ISO E SUPER® | 120,00 | 120,00 | 120,00 |
| KEPHALIS® | 5,00 | 5,00 | 5,00 |
| LINALOOL | 150,00 | 150,00 | 150,00 |
| LINALYLACETAT | 60,00 | 60,00 | 60,00 |
| BETA-METHYLNAPHTYLKETON | 5,00 | 5,00 | 5,00 |
| NEROLIDOL | 20,00 | 20,00 | 20,00 |
| NEROLIONE 10% in DPG | 20,00 | 20,00 | 20,00 |
| ORANGENÖL BRASILIANISCH | 100,00 | 100,00 | 100,00 |
| PHENYLETHYLACETAT | 5,00 | 5,00 | 5,00 |
| PHENYLETHYLALKOHOL | 30,00 | 30,00 | 30,00 |
| TERPINEOL | 20,00 | 20,00 | 20,00 |
| DIPROPYLENGLYCOL | 50,00 | 20,00 | 20,00 |
| MISCHUNG (**Mix1**) | - | 30,00 | - |
| MISCHUNG (**Mix2**) | - | - | 30,00 |
| TOTAL: | 1.000,00 | 1.000,00 | 1.000,00 |

### Parfümöl-Beispiel P4:

| | **Ref-P4** | **P4** | **P4a** |
|---|---|---|---|
| ALLYLAMYLGLYCOLAT | 10,00 | 10,00 | 10,00 |
| BERGAMOTTEÖL | 100,00 | 100,00 | 100,00 |
| CALONE® 1951 10% in DPG | 30,00 | 30,00 | 30,00 |
| CASSIS BASE | 10,00 | 10,00 | 10,00 |
| GEWÜRZNELKENÖL | 5,00 | 5,00 | 5,00 |
| ALPHA-DAMASCON 1 % in DPG | 20,00 | 20,00 | 20,00 |
| DIHYDROMYRCENOL | 60,00 | 60,00 | 60,00 |
| ETHYLENEBRASSYLAT (MACRO) | 20,00 | 20,00 | 20,00 |
| FARENAL® 1 % in DPG | 20,00 | 20,00 | 20,00 |
| FLORALOZONE® | 10,00 | 10,00 | 10,00 |
| GLOBALIDE® (MACRO) | 20,00 | 20,00 | 20,00 |
| HEDIONE® HC/30 | 10,00 | 10,00 | 10,00 |
| HEDIONE® | 200,00 | 200,00 | 200,00 |
| ALPHA-HEXYLZIMTALDEHYD | 100,00 | 100,00 | 100,00 |
| HEXYLSALICYLAT | 20,00 | 20,00 | 20,00 |
| ISO E SUPER® | 70,00 | 70,00 | 70,00 |
| KEPHALIS | 5,00 | 5,00 | 5,00 |
| LAVANDINÖL GROSSO NAT. | 20,00 | 20,00 | 20,00 |
| LEAFOVERT® 10% in DPG | 20,00 | 20,00 | 20,00 |
| LIGUSTRAL 10% in DPG | 10,00 | 10,00 | 10,00 |
| LILIAL® | 45,00 | 45,00 | 45,00 |
| LINALOOL | 60,00 | 60,00 | 60,00 |
| LINALYLACETAT | 50,00 | 50,00 | 50,00 |
| MANDARINALDEHYD 10% in TEC | 5,00 | 5,00 | 5,00 |
| ORANGENÖL BRASILIANISCH | 25,00 | 25,00 | 25,00 |
| POLYSANTOL® | 5,00 | 5,00 | 5,00 |
| TERPINEOL | 20,00 | 20,00 | 20,00 |
| DIPROPYLENGLYCOL | 30,00 | - | - |
| MISCHUNG (**Mix1**) | - | 30,00 | - |
| MISCHUNG (**Mix3**) | - | - | 30,00 |
| TOTAL: | 1.000,00 | 1.000,00 | 1.000,00 |

### Parfümöl-Beispiel P5:

| | **Ref-P5** | **P5** | **P5a** |
|---|---|---|---|
| Florazon | 15,00 | 15,00 | 15,00 |
| Cyclogalbanat® | 15,00 | 15,00 | 15,00 |
| Bergamotteöl | 135,00 | 135,00 | 135,00 |
| Mandarinenöl | 50,00 | 50,00 | 50,00 |
| Claritone® | 100,00 | 100,00 | 100,00 |
| Oxanthia 5% in DPG | 5,00 | 5,00 | 5,00 |
| Amarocit® | 10,00 | 10,00 | 10,00 |
| Pfefferminzöl | 5,00 | 5,00 | 5,00 |
| Artemisiaöl | 5,00 | 5,00 | 5,00 |
| Macisöl | 5,00 | 5,00 | 5,00 |
| Lilial® | 50,00 | 50,00 | 50,00 |
| Calone® 1951 10% in DPG | 25,00 | 25,00 | 25,00 |
| Geraniumöl | 10,00 | 10,00 | 10,00 |
| Hedione® | 240,00 | 240,00 | 240,00 |
| Hexenylsalicylat cis-3 | 15,00 | 15,00 | 15,00 |
| Parmanyl® | 5,00 | 5,00 | 5,00 |
| Beta-Ionon | 25,00 | 25,00 | 25,00 |
| Zedernholzöl (Cedarwood oil) | 30,00 | 30,00 | 30,00 |
| Iso E Super® | 80,00 | 80,00 | 80,00 |
| Vetival® | 40,00 | 40,00 | 40,00 |
| Vetikolacetat® | 5,00 | 5,00 | 5,00 |
| Sandranol® | 75,00 | 75,00 | 75,00 |
| Ysamber® K | 10,00 | 10,00 | 10,00 |
| Globalide® (MACRO) | 25,00 | 25,00 | 25,00 |
| Dipropylenglycol | 20,00 | 15,00 | 15,00 |
| MISCHUNG (**Mix1**) | - | 5,00 | - |
| MISCHUNG (**Mix2**) | - | - | 5,00 |
| TOTAL: | 1.000,00 | 1.000,00 | 1.000,00 |

### Parfümöl-Beispiel P6:

| | **Ref-P6** | **P6** | **P6a** |
|---|---|---|---|
| Leafovert® 10% in IPM | 10,00 | 10,00 | 10,00 |
| Bergamotteöl | 50,00 | 50,00 | 50,00 |
| Orangenöl | 40,00 | 40,00 | 40,00 |
| Oxanthia 10% in DPG | 5,00 | 5,00 | 5,00 |
| Öl von schwarzem Pfeffer 10% in DPG | 20,00 | 20,00 | 20,00 |
| Campher 10% in DPG | 5,00 | 5,00 | 5,00 |
| Gamma-Decalacton 10% in DPG | 15,00 | 15,00 | 15,00 |
| Gamma-Undecalacton 10% in DPG | 5,00 | 5,00 | 5,00 |
| Lilial® | 60,00 | 60,00 | 60,00 |
| Helional | 25,00 | 25,00 | 25,00 |
| Linalool | 80,00 | 80,00 | 80,00 |
| Geraniumöl 10% in DPG | 10,00 | 10,00 | 10,00 |
| Citronellol | 20,00 | 20,00 | 20,00 |
| Geraniol | 15,00 | 15,00 | 15,00 |
| Isodamascon® 10% in DPG | 20,00 | 20,00 | 20,00 |
| Hedione® | 120,00 | 120,00 | 120,00 |
| Alpha-Hexylzimtaldehyd | 40,00 | 40,00 | 40,00 |
| Vanillin | 15,00 | 15,00 | 15,00 |
| Coumarone® | 5,00 | 5,00 | 5,00 |
| Iso E Super® | 240,00 | 240,00 | 240,00 |
| Patchouliöl nat. | 25,00 | 25,00 | 25,00 |
| Sandalore | 10,00 | 10,00 | 10,00 |
| Labdanum Absolue 10% in DPG | 5,00 | 5,00 | 5,00 |
| Ysamber® K | 5,00 | 5,00 | 5,00 |
| Macrolide® Supra (MACRO) | 25,00 | 25,00 | 25,00 |
| Globalide® (MACRO) | 45,00 | 45,00 | 45,00 |
| Cyclohexadecanon (MACRO) | 15,00 | 15,00 | 15,00 |
| Ethylenbrassylat (MACRO) | 45,00 | 45,00 | 45,00 |
| Indole 10% in DPG | 5,00 | 5, 00 | 5,00 |
| Dipropylenglycol | 20,00 | 10,00 | 10,00 |
| MISCHUNG (**Mix1**) | - | 10,00 | - |
| MISCHUNG (**Mix2**) | - | - | 10,00 |
| TOTAL: | 1.000,00 | 1.000,00 | 1.000,00 |

### Parfümöl-Beispiel P7:

| | **Ref-P7** | **P7** | **P7a** |
|---|---|---|---|
| Styralylacetat | 10,00 | 10,00 | 10,00 |
| Linalylacetat | 40,00 | 40,00 | 40,00 |
| Mandaril 10% in DPG | 5,00 | 5,00 | 5,00 |
| Orangenöl | 60,00 | 60,00 | 60,00 |
| Oxanthia 5% in DPG | 5,00 | 5,00 | 5,00 |
| Gamma-Decalacton | 5,00 | 5,00 | 5,00 |
| Ethyl-3-methyl-3-phenylglycidat | 5,00 | 5,00 | 5,00 |
| Frambinon® | 5,00 | 5,00 | 5,00 |
| Himbeer-Base | 5,00 | 5,00 | 5,00 |
| Lilial® | 80,00 | 80,00 | 80,00 |
| Calone® 1951 10% in DPG | 20,00 | 20,00 | 20,00 |
| Majantol® | 80,00 | 80,00 | 80,00 |
| Linalool | 60,00 | 60,00 | 60,00 |
| Phenylethylalkohol | 20,00 | 20,00 | 20,00 |
| Citronellol | 20,00 | 20,00 | 20,00 |
| Geraniol | 5,00 | 5,00 | 5,00 |
| Benzylacetat | 30,00 | 30,00 | 30,00 |
| Hedione® | 150,00 | 150,00 | 150,00 |
| Alpha-Hexylzimtaldehyd | 150,00 | 150,00 | 150,00 |
| Benzylsalicylat | 60,00 | 60,00 | 60,00 |
| Amylsalicylat (n- und iso-) | 40,00 | 40,00 | 40,00 |
| Beta-Ionon | 20,00 | 20,00 | 20,00 |
| Eugenol | 5,00 | 5,00 | 5,00 |
| Ethylvanillin 10% in DPG | 10,00 | 10,00 | 10,00 |
| Zimtalkohol | 5,00 | 5,00 | 5,00 |
| Iso E Super® | 40,00 | 40,00 | 40,00 |
| Galaxolide® 50% in DEP (4,6,6,7,8,8-Hexamethyl-1,3,4,6,7,8-hexahydro-cyclopenta[g]benzopyran) (POLY) | 25,00 | 25,00 | 25,00 |
| Globalide® (MACRO) | 20,00 | 20,00 | 20,00 |
| Dipropylenglycol | 20,00 | 15,00 | 15,00 |
| MISCHUNG (**Mix2**) | - | 5,00 | - |
| MISCHUNG (**Mix3**) | - | - | 5,00 |
| TOTAL: | 1.000,00 | 1.000,00 | 1.000,00 |

### Parfümöl-Beispiel P8:

| | **Ref-P8** | **P8** | **P8a** |
|---|---|---|---|
| Limonenal 10% in DPG | 6,00 | 6,00 | 6,00 |
| Profarnesal 10% in DPG | 4,00 | 4,00 | 4,00 |
| Hexenylbutyrat cis-3 | 2,00 | 2,00 | 2,00 |
| Mintonat® | 3,00 | 3,00 | 3,00 |
| Linalylacetat | 120,00 | 120,00 | 120,00 |
| Citronenöl | 20,00 | 20,00 | 20,00 |
| Citrylal | 2,00 | 2,00 | 2,00 |
| Gamma-Nonalacton | 15,00 | 15,00 | 15,00 |
| Frambinon® | 2,00 | 2,00 | 2,00 |
| Himbeer-Base | 20,00 | 20,00 | 20,00 |
| Filbertone 1 % in DPG | 1,00 | 1,00 | 1,00 |
| Acetylmethylcarbinol, nat. 10% in DPG | 7,00 | 7,00 | 7,00 |
| Lilax Soft | 20,00 | 20,00 | 20,00 |
| Linalool | 30,00 | 30,00 | 30,00 |
| Terpinenol | 80,00 | 80,00 | 80,00 |
| Phenylethylalkohol | 20,00 | 20,00 | 20,00 |
| Geraniol | 40,00 | 40,00 | 40,00 |
| Nerol | 70,00 | 70,00 | 70,00 |
| Citronellol | 30,00 | 30,00 | 30,00 |
| Benzylacetat | 10,00 | 10,00 | 10,00 |
| Alpha-Amylzimtaldehyd | 60,00 | 60,00 | 60,00 |
| Hedione® | 130,00 | 130,00 | 130,00 |
| Beta-Ionon | 10,00 | 10,00 | 10,00 |
| Agrumex® HC | 80,00 | 80,00 | 80,00 |
| Oryclon® | 68,00 | 68,00 | 68,00 |
| Zedernholzöl (Cedarwood oil) | 2,00 | 2,00 | 2,00 |
| Vertofix® | 120,00 | 120,00 | 120,00 |
| Macrolide® (MACRO) | 20,00 | 20,00 | 20,00 |
| Triethylcitrat | 8,00 | 5,00 | 5,00 |
| MISCHUNG (**Mix3**) | - | 3,00 | - |
| MISCHUNG (**Mix1**) | - | - | 3,00 |
| TOTAL: | 1.000,00 | 1.000,00 | 1.000,00 |

### Parfümöl-Beispiel P9:

| | **Ref-P9** | **P9** | **P9a** |
|---|---|---|---|
| AMAROCIT® | 300,00 | 300,00 | 300,00 |
| AMBROXID | 150,00 | 150,00 | 150,00 |
| BENZYLSALICYLAT | 4.410,00 | 4.410,00 | 4.410,00 |
| CALONE® 1951 | 150,00 | 150,00 | 150,00 |
| MUSKATELLERSALBEIÖL | 60,00 | 60,00 | 60,00 |
| DAMASCON ALPHA, 10%ig in DPG | 70,00 | 70,00 | 70,00 |
| EVERNYL, 10% in DPG | 150,00 | 150,00 | 150,00 |
| GLOBANONE® | 1.200,00 | 1.200,00 | 1.200,00 |
| HELIONAL | 300,00 | 300,00 | 300,00 |
| HEXENYLSALICYLAT CIS-3 | 1.500,00 | 1.500,00 | 1.500,00 |
| ISOBUTYLCHINOLIN 1%ig in DPG | 150,00 | 150,00 | 150,00 |
| LILIAL® | 450,00 | 450,00 | 450,00 |
| POLYSANTOL | 60,00 | 60,00 | 60,00 |
| SYMMARINE® 1 %ig in DPG 10% DPG | 50,00 | 50,00 | 50,00 |
| MISCHUNG (**Mix2**) | - | 500,00 | - |
| MISCHUNG (**Mix3**) | - | - | 500,00 |
| TOTAL: | 9.000,00 | 9.500,00 | 9.500,00 |

### Parfümöl-Beispiel P10:

| FLORALOZONE | **Ref-P10** | **P10** | **P10a** |
|---|---|---|---|
| ALLYLAMYLGLYCOLAT | 2,00 | 2,00 | 2,00 |
| CYCLOGALBANAT® | 6,00 | 6,00 | 6,00 |
| MINTONAT | 4,00 | 4,00 | 4,00 |
| DIHYDROMYRCENOL | 60,00 | 60,00 | 60,00 |
| TERPINYLACETAT | 120,00 | 120,00 | 120,00 |
| CITRONENÖL | 60,00 | 60,00 | 60,00 |
| ORANGENÖL, | 20,00 | 20,00 | 20,00 |
| ARTEMISIA ÖL | 30,00 | 30,00 | 30,00 |
| BASILIKUMÖL | 2,00 | 2,00 | 2,00 |
| SCHWARZER PFEFFERÖL | 1,00 | 1,00 | 1,00 |
| ALDEHYD C14 SOGENANNT, 10%ig in DPG | 4,00 | 4,00 | 4,00 |
| FRUCTATE®, 10%ig in DPG | 1,50 | 1,50 | 1,50 |
| PEACHOLIDE, 10%ig in DPG | 0,50 | 0,50 | 0,50 |
| MUGETANOL | 4,00 | 4,00 | 4,00 |
| LINALOOL | 40,00 | 40,00 | 40,00 |
| GERANIUMÖL | 5,00 | 5,00 | 5,00 |
| HEXYLZIMTALDEHYD ALPHA | 30,00 | 30,00 | 30,00 |
| ETHYLVANILLIN | 1,00 | 1,00 | 1,00 |
| VANILLIN | 3,00 | 3,00 | 3,00 |
| COUMARIN | 20,00 | 20,00 | 20,00 |
| AGRUMEX | 1,50 | 1,50 | 1,50 |
| ZEDERNHOLZÖL | 20,00 | 20,00 | 20,00 |
| VERTOFIX | 40,00 | 40,00 | 40,00 |
| AMBERWOOD® F | 15,00 | 15,00 | 15,00 |
| CEDRAMBER | 30,00 | 30,00 | 30,00 |
| ISO E SUPER® | 180,00 | 180,00 | 180,00 |
| TRIMOFIX O | 10,00 | 10,00 | 10,00 |
| KOHINOOL | 10,00 | 10,00 | 10,00 |
| ISOLONGOFOLANOL | 5,00 | 5,00 | 5,00 |
| EBANOL | 5,00 | 5,00 | 5,00 |
| SANDALORE | 20,00 | 20,00 | 20,00 |
| SANDRANOL® | 60,00 | 60,00 | 60,00 |
| GUAIACHOLZÖL | 8,00 | 8,00 | 8,00 |
| AMBROCENIDE®, 10%ig in DPG | 2,00 | 2,00 | 2,00 |
| AMBROXIDE | 2,00 | 2,00 | 2,00 |
| YSAMBER® K | 18,00 | 18,00 | 18,00 |
| GLOBALIDE® (MACRO) | 24,00 | 24,00 | 24,00 |
| AURELIONE® (MACRO) | 16,00 | 16,00 | 16,00 |
| DPG | 18,00 | - | - |
| MISCHUNG (**Mix2**) | - | 18,00 | - |
| MISCHUNG (**Mix3**) | - | - | 18,00 |
| TOTAL: | 900,00 | 900,00 | 900,00 |

### Parfümöl-Beispiel P11:

| | **P11** | **P11a** |
|---|---|---|
| Acetophenon, 10%ig in DPG | 10,00 | 10,00 |
| n-Undecanal | 5,00 | 5,00 |
| Aldehyd C14 sogenannt (Pfirsichaldehyd) | 15,00 | 15,00 |
| Allylamylglycolat, 10%ig in DPG | 20,00 | 20,00 |
| Amylsalicylat | 25,00 | 25,00 |
| Benzylacetat | 60,00 | 60,00 |
| Citronellol | 80,00 | 80,00 |
| D-Limonen | 50,00 | 50,00 |
| Decenol trans-9 | 15,00 | 15,00 |
| Dihydromyrcenol | 50,00 | 50,00 |
| Dimethylbenzylcarbinylacetat | 30,00 | 30,00 |
| Diphenyloxid | 5,00 | 5,00 |
| Eucalyptol | 10,00 | 10,00 |
| Geraniol | 40,00 | 40,00 |
| Nerol | 20,00 | 20,00 |
| Geraniumöl | 15,00 | 15,00 |
| Hexenol cis-3, 10%ig in DPG | 5,00 | 5,00 |
| Hexenylsalicylat cis-3 | 20,00 | 20,00 |
| Indol, 10%ig in DPG | 10,00 | 10,00 |
| Alpha-Ionon | 15,00 | 15,00 |
| Isolongifolanol | 30,00 | 20,00 |
| Lilial (2-Methyl-3-(4-tert-butyl-phenyl)propanal) | 55,00 | 55,00 |
| Linalool | 40,00 | 40,00 |
| Methylphenylacetat | 10,00 | 10,00 |
| Phenylethylalkohol | 180,00 | 180,00 |
| Styralylacetat | 20,00 | 20,00 |
| Terpineol | 30,00 | 30,00 |
| Tetrahydrolinalool | 40,00 | 50,00 |
| Zimtalkohol | 10,00 | 10,00 |
| Macrolide (15-Pentadecanolid) (MACRO) | 115,00 | 100,00 |
| 5-Cyclohexadecen-1-on (MACRO) | - | 25,00 |
| MISCHUNG (**Mix2**) | 30,00 | - |
| MISCHUNG (**Mix3**) | - | 45,00 |
| Total: | 1.060,00 | 1.085,00 |

### Parfümöl-Beispiel P12:

| | **P12** | **P12a** |
|---|---|---|
| Benzylacetat | 60,00 | 60,00 |
| Citronellylacetat | 60,00 | 60,00 |
| Cyclamenaldehyd (2-Methyl-3-(4-isopropylphenyl)propanal | 20,00 | 20,00 |
| Dipropylenglykol | 100,00 | 60,00 |
| Ethyllinalool | 40,00 | 40,00 |
| Florol (2-Isobutyl-4-methyltetrahydro-2H-pyran-4-ol) | 30,00 | 30,00 |
| Globanone [(E/Z)-8-Cyclohexadecen-1-on] (MACRO) | 180,00 | 180,00 |
| Hedione (Methyldihydrojasmonat) | 140,00 | 140,00 |
| Hexenylsalicylat, cis-3 | 10,00 | 10,00 |
| Vertocitral (2,4-dimethyl-3-cyclohexencarboxaldehyd) | 5,00 | 5,00 |
| Hydratropaaldehyd, 10%ig in DPG | 5,00 | 5,00 |
| Isodamascon (1-(2,4,4-Trimethyl-2-cyclohexen-1-yl)-2-buten-1-on), 10%ig in DPG | 5,00 | 5,00 |
| Isomuscone (Cyclohexadecanon) (MACRO) | 40,00 | 40,00 |
| Jacinthaflor (2-Methyl-4-phenyl-1,3-dioxolan) | 10,00 | 10,00 |
| Cis-Jasmon, 10%ig in DPG | 20,00 | 20,00 |
| Linalool | 75,00 | 75,00 |
| Linalylacetat | 30,00 | 30,00 |
| Methylbenzoat, 10%ig in DPG | 25,00 | 25,00 |
| para-Methylkresol, 10%ig in DPG | 10,00 | 10,00 |
| Nerol | 40,00 | 40,00 |
| Phenylpropylaldehyd | 5,00 | 5,00 |
| 2-Phenylethylalkohol | 80,00 | 80,00 |
| Tetrahydrogeraniol | 30,00 | 20,00 |
| 2,2-Dimethyl-3-cyclohexyl-1-propanol | 10,00 | 20,00 |
| MISCHUNG (**Mix2**) | 20,00 | 40,00 |
| Total: | 1.050,00 | 1.030,00 |

Die erfindungsgemäßen Parfümöle P1, P2, P3, P4, P5, P6, P7, P8, P9, P10, P11 bzw. P12 aus den obigen Parfümöl-Beispielen P1 bis P12 wurden jeweils separat in die nachfolgenden Formulierungen eingearbeitet.

Die bei dem jeweiligen Parfümöl beobachteten geruchlichen Effekte wurden jeweils auch in den nachfolgenden Formulierungen beobachtet.

### Formulierungsbeispiele

### Beispiel F1 - Waschpulver

| **Material** | **Chemischer Name** | **Funktion** | **Gew.-%** | **Gew.-%** |
|---|---|---|---|---|
| Natrium Metasilicat Pentahydrat | Sodium Metasilicate Pentahydrate | | Ad 100 | Ad 100 |
| Natriumhydrogencarbonat | Sodium hydrogen carbonate | Alkali | 15,0 | 15,0 |
| Natriumpercarbonat | Sodium carbonate peroxyhydrate | Bleichmittel | 15,0 | 15,0 |
| Peractive AC Blue | TAED / Na-Carboxymethylcellulose | Aktivator | 5,00 | 5,00 |
| Genapol OA-080 | Oxoalkohol C14-15, 8EO | Nichtionisches Tensid | 3,00 | 3,00 |
| Texapon K12 Pulver | Sodium Lauryl Sulphate C12 | Anionisches Tensid | 7,00 | 7,00 |
| Tinopal CBS-X | | Aufheller | 0,50 | 0,50 |
| Savinase 6.0 T, Type W | Protease | Enzym | 0,40 | 0,40 |
| Termamyl 120 T | Alpha-Amylase | Enzym | 0,30 | 0,30 |
| Natriumsulfat | Sodium Sulphate | Füllstoff | 5,50 | 5,50 |
| Parfumöl P1, P2, P3, P4, P5, P6, P7, P8, P9, P10, P11 bzw. P12 | | Parfum (Fragrance) | 0,30 | 0,50 |

### Beispiel F2 - Allzweckreiniger

| **Material** | **Chemischer Name** | **Funktion** | **Gew.-%** | **Gew.-%** |
|---|---|---|---|---|
| Entionisiertes Wasser | Water | Lösungsmittel | Ad 100 | Ad 100 |
| Mergal K9N | 5-Chloro-2-methyl-3-(2H)-isothiazolone und 2-methyl-3-(2H)-isothiazolone | Konservierungsmittel | 0,1 | 0,1 |
| Trinatriumcitrat Dihydrat | Tri Sodium Citrate Dihydrate | Komplexbildner | 3,0 | 3,0 |
| Zetesol NL-2 | Fatty alcohol C12-14-sulfate, Sodium | Anionisches Tensid | 30,0 | 30,0 |
| Imbentin C/125/055 | Fatty alcohol C12-C15, 8EO | Nichtionisches Tensid | 5,0 | 5,0 |
| Ethanol | Ethanol | Lösungsmittel | 2,0 | 2,0 |
| Parfumöl P1, P2, P3, P4, P5, P6, P7, P8, P9, P10, P11 bzw. P12 | | Parfum (Fragrance) | 0,3 | 0,5 |

### Beispiel F3 - Shampoo

| **Material** | **INCI-Name** | **Gew.-%** | **Gew.-%** |
|---|---|---|---|
| Entionisiertes Wasser | Water | Ad 100 | Ad 100 |
| Plantacare PS 10 | Sodium Laureth Sulfate, Lauryl Glucoside | 20,0 | 20,0 |
| Euperlan PK 771 | Glycol Distearate, Sodium Lauryl Sulfate, Cocamide MEA, Laureth-10 | 6,0 | 6,0 |
| Dragocid Liquid | Phenoxyethanol, Methylparaben, Ethylparaben, Butylparaben, Propylparaben, Isobutylparaben | 0,5 | 0,5 |
| Natrium Chlorid | Sodium Chloride | 1,4 | 1,4 |
| Citronensäure Monohydrat kristallin | Citric Acid | 0,1 | 0,1 |
| Parfumöl P1, P2, P3, P4, P5, P6, P7, P8, P9, P10, P11 bzw. P12 | Parfum (Fragrance) | 0,5 | 0,8 |

### Beispiel F4 - Duschgel

| **Material** | **INCI-Name** | **Gew.-%** | **Gew.-%** |
|---|---|---|---|
| Entionisiertes Wasser | Wasser | Ad 100 | Ad 100 |
| Plantacare PS 10 | Sodium Laureth Sulfate, Lauryl Glucoside | 20,0 | 20,0 |
| Dragocid Liquid | Phenoxyethanol, Methylparaben, Ethylparaben, Butylparaben, Propylparaben, Isobutylparaben | 0,5 | 0,5 |
| Natriumchlorid | Sodium Chloride | 1,4 | 1,4 |
| Citronensäure Monohydrat kristallin | Citric Acid | 1,3 | 1,3 |
| Parfumöl P1, P2, P3, P4, P5, P6, P7, P8, P9, P10, P11 bzw. P12 | Parfum (Fragrance) | 0,5 | 0,7 |

### Beispiel F5 - Weichspüler

| **Material** | **Chemischer Name** | **Funktion** | **Gew.-%** | **Gew.-%** |
|---|---|---|---|---|
| Entionisiertes Wasser | Wasser | Lösungsmittel | Ad 100 | Ad 100 |
| Rewoquat WE 18 | Dialkylesterammoniumethosulfate | Kationisches Tensid | 16,6 | 16,6 |
| Mergal K9N | 5-Chloro-2-methyl-3-(2H)-isothiazolone und 2-methyl-3-(2H)-isothiazolone | Konservierungsmittel | 0,10 | 0,10 |
| Dow Corning 1520 Antifoam | Polydimethyl-siloxane | Entschäumer | 0,30 | 0,30 |
| Magnesiumchlorid 1%ige Lösung | Magnesiumchlorid Lösung | Konsistenzgeber | 10,00 | 10,00 |
| Parfumöl P1, P2, P3, P4, P5, P6, P7, P8, P9, P10, P11 bzw. P12 | | Fragrance | 0,55 | 0,75 |

### Beispiel F6 - Eau de Cologne / Eau de Toilette

| **Inhaltsstoffe** | **Gew.-%** | **Gew.-%** |
|---|---|---|
| Parfumöl P1, P2, P3, P4, P5, P6, P7, P8, P9, P10, P11 bzw. P12 | 5 | 10 |
| Ethanol | Ad 100 | Ad 100 |
| Farbstoff, UV-Stabilisator | q.s. | q.s. |
| Wasser | 18 | 10 |

### Beispiel F7 - Aerosol-Pumpspray

| **Inhaltsstoffe** | **Gew.-%** | **Gew.-%** |
|---|---|---|
| Parfumöl P1, P2, P3, P4, P5, P6, P7, P8, P9, P10, P11 bzw. P12 | 1,0 | 1,5 |
| Ethanol | Ad 100 | Ad 100 |
| Wasser | 5,0 | 8,0 |
| Alpha-Tocopherol | 0,20 | 0,20 |
| Hydroxypropylcellulose | 0,20 | - |
| Rosmarinextrakt, in Ethanol löslich | 0,22 | - |
| Cetylalkohol | 1,00 | 0,50 |

### Beispiel F8 - Shampoo

| **Inhaltsstoffe** | **Gew.-%** | **Gew.-%** | **Gew.-%** |
|---|---|---|---|
| Natriumlaurylethersulfat (z.B. Texapon NSO) | 12 | 12 | 12 |
| Cocamidopropylbetain (z.B. Dehyton K) | 2 | 2 | 2 |
| Natriumchlorid | 1,4 | 1,4 | 1,4 |
| Citronensäure | 1,3 | 1,3 | 1,3 |
| Phenoxyethanol, Methyl-, Ethyl-, Butyl- und Propylparaben | 0,5 | 0,5 | 0,5 |
| Parfumöl P1, P2, P3, P4, P5, P6, P7, P8, P9, P10, P11 bzw. P12 | 0,3 | 0,5 | 0,7 |
| Wasser | Ad 100 | Ad 100 | Ad 100 |

### Beispiel F9 - Waschpulver

| **Inhaltsstoffe** | **Gew.-%** | **Gew.-%** | **Gew.-%** |
|---|---|---|---|
| Lineares Na-Alkylbenzolsulfonat | 8,8 | 8,8 | 8,8 |
| Ethoxylierter Fettalkohol C12-18 (7 EO) | 4,7 | 4,7 | 4,7 |
| Na-Seife | 3,2 | 3,2 | 3,2 |
| Entschäumer DOW CORNING(R) 2-4248S POWDERED ANTIFOAM, Silikonöl auf Zeolith als Trägermaterial | 3,9 | 3,9 | 3,9 |
| Zeolith 4A | Ad 100 | Ad 100 | Ad 100 |
| Na-Carbonat | 11,6 | 11,6 | 11,6 |
| Na-Salz eines Copolymers aus Acryl- und Maleinsäure (Sokalan CP5) | 2,4 | 2,4 | 2,4 |
| Na-Silicat | 3,0 | 3,0 | 3,0 |
| Carboxymethylcellu lose | 1,2 | 1,2 | 1,2 |
| Dequest 2066([[(Phosphonomethyl)imino]bis[(ethylennitrilo)bis (methylen)]]tetrakis-phosphonsäure, Natriumsalz) | 2,8 | 2,8 | 2,8 |
| Optischer Aufheller | 0,2 | 0,2 | 0,2 |
| Na-Sulfat | 6,5 | 6,5 | 6,5 |
| Protease | 0,4 | 0,4 | 0,4 |
| Natriumperborat tetrahydrat | 21,7 | 21,7 | 21,7 |
| Parfumöl P1, P2, P3, P4, P5, P6, P7, P8, P9, P10, P11 bzw. P12 | 0,25 | 0,35 | 0,5 |
| EDTA | 1,0 | 1,0 | 1,0 |

### Beispiel F10 - Flüssigwaschmittel

| **Inhaltsstoffe** | **Gew.-%** |
|---|---|
| Entionisiertes Wasser | 39,9 |
| Optischer Aufheller | 0,10 |
| Kokos Fettsäuren (C12-C18) | 7,5 |
| Kaliumhydroxid 50%ige Lösung | 4,3 |
| Propan-1,2-diol | 5,00 |
| Fettalkohole C12-C15, 8 EO | 12,0 |
| Na-Salz sekundärer Alkylsulfonate (C13-C17) | 17,0 |
| Triethanolamin | 2,0 |
| Trinatriumcitrat Dihydrat | 5,0 |
| Dequest 2066([[(Phosphonomethyl)imino]bis[(ethylennitrilo)bis (methylen)]]tetrakis-phosphonsäure, Natriumsalz) | 3,0 |
| Ethanol | 3,0 |
| Enzyme | 0,7 |
| Parfumöl P1, P2, P3, P4, P5, P6, P7, P8, P9, P10, P11 bzw. P12 | 0,5 |

### Beispiel F11 - Flüssigwaschmittel Konzentrat

| **Inhaltsstoffe** | **Gew.-%** |
|---|---|
| Entionisiertes Wasser | 13,4 |
| Kokos Fettsäuren (C12-C18) | 10,0 |
| Fettalkohole C12-C15, 8 EO | 26,0 |
| Na-Salz sekundärer Alkylsulfonate (C13-C17) | 26,5 |
| Triethanolamin | 8,5 |
| Na-Salz von Fettalkoholsulfaten C12-C14 | 3,0 |
| Ethanol | 5,5 |
| Harnstoff | 4,5 |
| Enzyme | 0,9 |
| Citronensäure | 1,0 |
| Parfumöl P1, P2, P3, P4, P5, P6, P7, P8, P9, P10, P11 bzw. P12 | 0,7 |

### Beispiel F12: Deo-Formulierung in Form eines Roll-on-Gels

| Komponente / NAME | Gew.-% | Gew.-% |
|---|---|---|
| 1,3-Butylenglykol | 2,00 | 2,00 |
| PEG-40-Hydriertes Rizinusöl | 2,00 | 2,00 |
| Hydroxyethylcellulose | 0,50 | 0,50 |
| Parfumöl P1, P2, P3, P4, P5, P6, P7, P8, P9, P10, P11 bzw. P12 | 0,60 | 0,75 |
| Phenoxyethanol (Konservierungsstoff) | 0,30 | - |
| Wasser | ad 100,00 | ad 100,00 |

### Beispiel F13: Creme

| Komponente / NAME | Gew.-% | Gew.-% |
|---|---|---|
| Paraffinöl | 10,00 | 10,00 |
| Ozokerit | 4,00 | 4,00 |
| Vaseline | 4,00 | 4,00 |
| pflanzliches Öl | 10,00 | 10,00 |
| Wollwachsalkohol | 2,00 | 2,00 |
| Aluminiumstearat | 0,40 | 0,40 |
| Parfumöl P1, P2, P3, P4, P5, P6, P7, P8, P9, P10, P11 bzw. P12 | 0,80 | 1,00 |
| 1,2-Pentandiol | 2,00 | 2,00 |
| Wasser | ad 100,00 | ad 100,00 |

### Beispiel F14: O/W-Lotion

| Komponente / NAME | Gew.-% | Gew.-% |
|---|---|---|
| Paraffinöl | 5,00 | 5,00 |
| Isopropylpalmitat | 5,00 | 5,00 |
| Cetylalkohol | 2,00 | 2,00 |
| Bienenwachs | 2,00 | 2,00 |
| Ceteareth-20 | 2,00 | 2,00 |
| PEG-20-Glycerylstearat | 1,50 | 1,50 |
| Glycerin | 3,00 | 3,00 |
| Parfumöl P1, P2, P3, P4, P5, P6, P7, P8, P9, P10, P11 bzw. P12 | - | 0,80 |
| Parabene | 0,30 | 0,30 |
| Wasser | ad 100,00 | ad 100,00 |

### Beispiel F15: Raumluftverbesserer (Air Freshener) in Gelform

| Komponente / NAME | Gew.-% | Gew.-% |
|---|---|---|
| Entmineralisiertes Wasser | Ad 100,00 | Ad 100,00 |
| Genugel^{®} X-6424 (1) (Carrageenan) | 2,00 | 2,00 |
| Parfumöl P1, P2, P3, P4, P5, P6, P7, P8, P9, P10, P11 bzw. P12 | 0,80 | 1,20 |
| Arkopal^{®} N 100 oder Tergitol^{®} NP 10 (Emulgator) | 3,50 | 3,50 |
| Preventol^{®} D (Konservierungsmittel) | 5,00 | 5,00 |

### Beispiel F16: Air-freshener in Gelform

5 g Accurel (poröses Homo-Polypropylen Pulver mit 75% Hohlraumanteil, Produkt der Akzo Nobel Faser AG, Obernburg, Deutschland) werden mit 15 g des Parfümöls Parfumöl P1, P2, P3, P4, P5, P6, P7, P8, P9, P10, P11 bzw. P12 durch Mischen der Bestandteile unter Vakuum beladen. Das resultierende Pulver wird anschließend bei Normaldruck mit 4,5 g Wasser verrührt, wodurch Mischung M1 erhalten wird. In einem separaten Gefäß werden 2,5 g Carrageenan, 0,3 g Chloracetamid und 0,5 g Calciumchlorid-Dihydrat in 62 g Wasser unter Erwärmen bei maximal 75 °C gelöst. In diese Lösung wird unter Rühren Mischung M1 eingebracht und homogenisiert. Die resultierende, vorzugsweise noch warme Mischung wird in die gewünschte Form (Kugeln, Halbkugeln, Kissen, Zylinder, Quader, Würfel, Muscheln oder ähnliche) gegossen. Nach dem Abkühlen auf etwa 20°C werden Raumerfrischer in Gelform erhalten, deren Beladung an jeweiligem Parfümöl bei etwa 20 Gew.-% bezogen auf das Gesamtgewicht des Raumerfrischers liegt.

### Beispiel F17: Haarfestiger

| Komponente / NAME | Gew.-% | Gew.-% |
|---|---|---|
| Polyvinylpyrrolidon/Vinylacetat/ Vinylpropionat-Copolymer | 5,00 | 5,00 |
| Ethanol | 45,00 | 45,00 |
| Parfumöl P1, P2, P3, P4, P5, P6, P7, P8, P9, P10, P11 bzw. P12 | 0,50 | 0,60 |
| Konservierungsstoffe | 1,50 | 1,50 |
| Wasser | ad 100,00 | ad 100,00 |

### Beispiel F18: Pflegemaske

| Komponente / NAME | Gew.-% | Gew.-% |
|---|---|---|
| PEG-50 Lanolin | 0,50 | 0,50 |
| Glycerylstearat | 2,00 | 2,00 |
| Sonnenblumenkernöl | 3,00 | 3,00 |
| Bentonit | 8,00 | 8,00 |
| Kaolin | 35,00 | 35,00 |
| Zinkoxid | 5,00 | 4,00 |
| Parfumöl P1, P2, P3, P4, P5, P6, P7, P8, P9, P10, P11 bzw. P12 | 0,35 | 0,50 |
| Konservierungsstoffe | q.s. | q.s. |
| Wasser | ad 100,00 | ad 100,00 |

### Beispiel F19: Hautöl

| Komponente / NAME | Gew.-% | Gew.-% |
|---|---|---|
| Cetylpalmitat | 3,00 | 3,00 |
| C₁₂₋₁₅- Alkylbenzoat | 2,00 | 2,00 |
| Polyisobuten | 10,00 | 10,00 |
| Squalan | 2,00 | 2,00 |
| Parfumöl P1, P2, P3, P4, P5, P6, P7, P8, P9, P10, P11 bzw. P12 | 0,50 | 0,40 |
| Konservierungsstoffe | q.s. | q.s. |
| Paraffinöl | ad 100,00 | ad 100,00 |

### Beispiel F20: Haarconditioner mit UV-Schutz

| Komponente | INCI Name | Gew.-% | Gew.-% |
|---|---|---|---|
| Renex PEG 6000 | PEG-150 | 2,50 | 2,50 |
| Hair Conditioner Base | Cetyl Alcohol, Behentrimonium Chloride, Triticum Vulgare (Wheat) Bran Extract, Linoleic Acid | 3,00 | 3,00 |
| PCL-Solid | Stearyl Heptanoate, Stearyl Caprylate | 0,50 | 0,50 |
| Dow Corning 5200 | Laurylmethicone Copolyol | 0,50 | 0,50 |
| Natrosol 250 HR | Hydroxyethylcellulose | 0,50 | 0,50 |
| Benzophenon-4 | Benzophenone-4 | 1,00 | 0,50 |
| Neo Heliopan AP | Disodium Phenyl Dibenzimidazole Tetrasulfonate | 1,00 | 0,80 |
| Amino Methyl propanol | Amino Methyl propanol | 2,00 | 1,20 |
| Nipagin M | Methylparaben | 0,30 | 0,30 |
| Dow Corning 949 Cationic Emulsion | Amodimethicone, Cetrimonium Chloride, Trideceth-12 | 2,00 | 2,00 |
| Parfumöl P1, P2, P3, P4, P5, P6, P7, P8, P9, P10, P11 bzw. P 12 | Parfum | 0,80 | 1,00 |
| Wasser | Water (Aqua) | Ad 100 | Ad 100 |

### Beispiel F21: Deostifte

| Komponente / NAME | Gew.-% | Gew.-% |
|---|---|---|
| Natriumstearat | 8,00 | 8,00 |
| PPG-3 Myristyl ether | 70,00 | 70,00 |
| 1,2-Propylenglykol | 10,00 | 10,00 |
| 1,1-Dimethyl-3-phenylpropanol | 0,20 | - |
| 2-Butyloctansäure | - | 0,20 |
| Parfumöl P1, P2, P3, P4, P5, P6, P7, P8, P9, P10, P11 bzw. P12 | 0,55 | 0,65 |
| Wasser | Ad 100 | Ad 100 |

### Beispiel F22: Mikroemulsionsgele

| Komponente / NAME | Gew.-% | Gew.-% |
|---|---|---|
| Glycerinisostearat | 1,80 | 2,00 |
| Octoxyglycerin | 1,00 | 0,80 |
| Ceteareth-15 | 5,20 | 5,00 |
| PEG-150 Distearat | 1,00 | 1,00 |
| Aluminiumchlorohydrat | 5,00 | 5,00 |
| Isotridecylisononanoat | 3,30 | 3,50 |
| Cyclomethicon | 6,60 | 6,40 |
| Parfumöl P1, P2, P3, P4, P5, P6, P7, P8, P9, P10, P11 bzw. P 12 | 0,60 | 0,70 |
| Wasser | Ad 100 | Ad 100 |

### Beispiel F23: Antiperspirant-Formulierungen

| Komponente / NAME | Gew.-% | Gew.-% |
|---|---|---|
| Reach AZP-908 SUF (Aluminum-Zirconium TetrachlorohydrexGly) | 24,00 | 22,00 |
| Cyclomethicone (Pentamer) | Ad 100 | Ad 100 |
| Polydecen (Silkflo 364 NF) | 17,50 | 20,00 |
| Neo Heliopan OS (Ethylhexyl Salicylate, Symrise) | 2,50 | 1,00 |
| L-Menthyllactat (Frescolat ML, Symrise) | 0,25 | - |
| Polyethylen | 3,00 | 3,00 |
| Hydriertes Rizinusöl | 2,00 | 2,00 |
| Promyristyl PM-3 | 7,00 | 7,00 |
| PEG-8 Distearat | 3,00 | 3,00 |
| Siliciumdioxid (Cab-O-Sil M-5) | 1,00 | 1,00 |
| Stearylalkohol | 15,00 | 10,00 |
| Octyldodecanol | - | 8,00 |
| Parfumöl P1, P2, P3, P4, P5, P6, P7, P8, P9, P10, P11 bzw. P12 | 0,75 | 0,95 |

### Beispiel F24: Suspensionssticks

| Komponente / NAME | Gew.-% | Gew.-% | Gew.-% |
|---|---|---|---|
| Stearylalkohol | 20,00 | 20,00 | 20,00 |
| Cyclomethicone | Ad 100 | Ad 100 | Ad 100 |
| PPG-14 Butylether | 2,00 | 2,00 | 2,00 |
| Hydriertes Rizinusöl | 1,00 | 1,00 | 1,00 |
| Talk | 2,00 | 2,00 | 2,00 |
| Aluminium Chlorhydrat, Pulver | 20,00 | 20,00 | 20,00 |
| Triclosan® (5-Chlor-2-(2,4-dichlorphenoxy)phenol) | 0,30 | - | 0,30 |
| Ethylhexylglycerin (Octoxyglycerin) | 0,50 | 0,80 | 0,50 |
| 1,1-Dimethyl-3-phenylpropanol | 0,30 | 0,25 | 0,35 |
| Anisalkohol | - | - | 0,15 |
| Parfumöl P1, P2, P3, P4, P5, P6, P7, P8, P9, P10, P11 bzw. P12 | 0,90 | 0,85 | 0,75 |

### Beispiel F25: Deo Zerstäuber

| Komponente / NAME | Gew.-% | Gew.-% |
|---|---|---|
| PEG-40-Hydriertes Rizinusöl | 3,00 | 3,00 |
| Ethylhexylglycerin (Octoxyglycerin) | 0,80 | 0,80 |
| Ethanol | 40,00 | 40,00 |
| Citrat-Puffer | 0,50 | 0,50 |
| 1,2-Hexandiol/1,2-Octandiol (1:1) | 0,50 | 0,25 |
| Phenoxyethanol | | 0,35 |
| Triclosan® (5-Chlor-2-(2,4-dichlorphenoxy)phenol) | 0,25 | - |
| 2-Benzylheptan-1-ol (Jasmol) | - | 0,05 |
| Parfumöl P1, P2, P3, P4, P5, P6, P7, P8, P9, P10, P11 bzw. P12 | 0,90 | 0,75 |
| Wasser | Ad 100 | Ad 100 |

## Patentansprüche

1. Riechstoffmischung, vorzugsweise Parfümöl, umfassend
(a) Verbindung **(A-1)** und Verbindung (**A-2**) 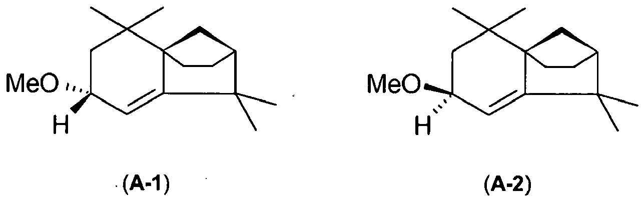 und
(b) einen oder mehrere weitere Moschusriechstoffe, welche keine Isolongifolenylmethylether sind,
wobei vorzugsweise das Massenverhältnis der Gesamtmenge an Moschusriechstoffen des Bestandteils (b) zu der Gesamtmenge der Verbindungen (**A-1**) und (**A-2**) (Bestandteil (a)) größer oder gleich 1 : 2 ist, bevorzugt größer oder gleich 1 : 1, weiter bevorzugt größer oder gleich 3 : 2.

2. Riechstoffmischung, vorzugsweise Parfümöl, nach Anspruch 1, wobei das Massenverhältnis der Gesamtmenge an Moschusriechstoffen des Bestandteils (b) zu der Gesamtmenge der Verbindungen des Bestandteils (a) im Bereich von 3 : 2 bis 20 : 1 liegt, bevorzugt im Bereich von 3 : 2 bis 15 : 1, besonders bevorzugt im Bereich von 2 : 1 bis 12 : 1.

3. Riechstoffmischung, vorzugsweise Parfümöl, nach einem der vorhergehenden Ansprüche, wobei der oder die weiteren Moschusriechstoffe des Bestandteils (b) gewählt sind aus der Gruppe der makrocyclischen Moschusriechstoffe, der Nitro-Moschusriechstoffe, der polycyclischen Moschusriechstoffe und/oder der alicyclischen Moschusriechstoffe.

4. Riechstoffmischung, vorzugsweise Parfümöl, nach einem der vorhergehenden Ansprüche, wobei das Massenverhältnis der Gesamtmenge des Isomers (**A-1**) zu der Gesamtmenge des Isomers (**A-2**) größer oder gleich 11 : 4 ist, bevorzugt größer oder gleich 3 : 1, weiter bevorzugt größer oder gleich 7 : 2, und bevorzugt im Bereich von 3 : 1 bis 12 : 1 liegt, weiter bevorzugt im Bereich von 7 : 2 bis 10 : 1, besonders bevorzugt im Bereich von 15 : 4 bis 10 : 1, und am meisten bevorzugt im Bereich von 4 :1 bis 8 : 1.

5. Riechstoffmischung, vorzugsweise Parfümöl, nach einem der vorhergehenden Ansprüche, enthaltend zusätzlich
(c) einen, 2, 3, 4, 5 oder mehr Terpenalkohole mit der Summenformel C₁₀HₓO, wobei x die Zahl 18, 20 oder 22 bedeutet, und wobei vorzugsweise ein, mehrere oder sämtliche Terpenalkohole ausgewählt sind aus der Gruppe bestehend aus Linalool, Eucalyptol, Dihydromyrcenol, Citronellol, Nerol, Geraniol, Menthol, Tetrahydrolinalool und Tetrahydrogeraniol,
und/oder
(d) einen, 2, 3, oder mehr weitere holzige Riechstoffe, wobei der weitere holzige Riechstoff oder die weiteren holzigen Riechstoffe des Bestandteils (d) von Bestandteil (a) und Bestandteil (b) verschieden ist bzw. sind.

6. Riechstoffmischung, vorzugsweise Parfümöl, nach Anspruch 5, wobei das Massenverhältnis der Gesamtmenge an Terpenalkoholen (Bestandteil (c)) zu der Gesamtmenge an Verbindungen (**A-1**) und (**A-2**) (Bestandteil (a)) größer oder gleich 1 : 1 ist, bevorzugt größer oder gleich 2 : 1, weiter bevorzugt größer oder gleich 3 : 1, besonders bevorzugt größer oder gleich 4 : 1.

7. Riechstoffmischung, vorzugsweise Parfümöl, nach einem der vorhergehenden Ansprüche, wobei Bestandteil (d) einen, zwei, drei oder mehrere weitere holzige Riechstoffe umfasst oder daraus besteht, ausgewählt aus der Gruppe bestehend aus
(d)(i) 2,3,8,8-Tetramethyl-1,2,3,4,5,6,7,8-octahydro-2-naphthalenylmethylketon und/oder 1-[1,2,3,4,6,7,8,8a-octahydro-1,2,8,8-tetramethylnaphtalen-2-yl]ethanon
und/oder
(d)(ii) Sandelholzriechstoffen, vorzugsweise ausgewählt aus der Gruppe bestehend aus 2-Methyl-4-(2,2,3-trimethyl-3-cyclopenten-1-yl)butanol, 5-(2,2,3-Trimethyl-3-cyclopenten-1-yl)-3-methylpentan-2-ol, 2-Ethyl-4-(2,2,3-trimethyl-3-cydopenten-1-yl)-2-buten-1-ol, 3-Methyl-5-(2,2,3-trimethyl-3-cyclopenten-1-yl)-4-penten-2-ol, 3,3-Dimethyl-5-(2,2,3-trimethyl-3-cyclopenten-1-yl)-4-penten-2-ol, 3-Isocamphylcyclohexanol und [1-Methyl-2-(1,2,2-trimethyl[3.1.0]hex-3-ylmethyl)cyclopropyl]methanol.

8. Riechstoffmischung, vorzugsweise Parfümöl, nach Anspruch 7, wobei das Massenverhältnis der Gesamtmenge an Bestandteil (d)(i) 2,3,8,8-Tetramethyl-1,2,3,4,5,6,7,8-octahydro-2-naphthalenylmethylketon und 1-[1,2,3,4,6,7,8,8a-octahydro-1,2,8,8-tetramethylnaphtalen-2-yl]ethanon zu der Gesamtmenge an Verbindungen (**A-1**) und (**A-2**) (Bestandteil (a)) größer oder gleich 1 : 1 ist, bevorzugt größer oder gleich 2 : 1, weiter bevorzugt größer oder gleich 3 : 1, besonders bevorzugt größer oder gleich 4 : 1.

9. Riechstoffmischung, vorzugsweise Parfümöl, nach Anspruch 7 oder 8, wobei das Massenverhältnis der Gesamtmenge an Sandelholzriechstoffen (Bestandteil (d)(ii)) zu der Gesamtmenge an Verbindungen (**A-1**) und (**A-2**) (Bestandteil (a)) größer oder gleich 1 : 1 ist, vorzugsweise größer oder gleich 2 : 1, bevorzugt größer oder gleich 3 : 1, weiter bevorzugt größer oder gleich 4 : 1.

10. Riechstoffmischung, vorzugsweise Parfümöl, nach einem der vorhergehenden Ansprüche, wobei Bestandteil
(b) einen oder mehrere makrocyclische Moschusriechstoffe enthält, vorzugsweise gewählt aus der Gruppe bestehend aus makrocyclischen C₁₄-C₁₈-Ketonen und makrocyclischen C₁₄-C₁₈-Lactonen, wobei das Keton bzw. Lacton vorzugsweise eine Ringgröße von 15 bis 17 Ringatomen und kein, ein oder zwei Sauerstoffatome im Ring aufweist.

11. Riechstoffmischung, vorzugsweise Parfümöl, nach einem der vorhergehenden Ansprüche, wobei Bestandteil
(b) 15-Pentadec-(11/12)-enolid, Ethylenbrassylat, 15-Pentadecanolid, 5-Cyclohexadecen-1-on, 7-Cyclohexadecen-1-on, 8-Cyclohexadecen-1-on, 7/8-Cyclohexadecen-1-on, Cyclohexadecanon, 3-Methylcyclopentadecanon, 3-Methylcyclopentadecenon und/oder 9-Cycloheptadecen-1-on,
ist oder enthält.

12. Parfümiertes Produkt enthaltend eine Riechstoffmischung, vorzugsweise ein Parfümöl, nach einem der vorhergehenden Ansprüche, vorzugsweise in einer sensorisch wirksamen Menge.

13. Parfümiertes Produkt nach Anspruch 12, ausgewählt aus der Gruppe bestehend aus Wasch- und Reinigungsmitteln und Hygiene- und Pflegeprodukten, insbesondere im Bereich der Körperpflege, der Haarpflege, der Kosmetik und des Haushalts.

14. Riechstoffmischung nach einem der Ansprüche 1 bis 11 oder parfümiertes Produkt nach Anspruch 12 oder 13, **dadurch gekennzeichnet, dass** die Gesamtmasse der Verbindungen (**A-1**) und (**A-2**) (Bestandteil (a)) im Bereich von 0,25 bis 10 Gew.-% liegt, bevorzugt im Bereich von 0,5 bis 8 Gew.-%, besonders bevorzugt im Bereich von 0,75 bis 7,5 Gew.-%, insbesondere bevorzugt im Bereich von 1 bis 6 Gew.-%, jeweils bezogen auf die Gesamtmasse aller in der Riechstoffmischung bzw. dem parfümierten Produkt enthaltenen Riechstoffe.

15. Verfahren zum Modifizieren, vorzugsweise zum Verringern oder Unterdrücken einer metallischen Note einer oder mehrerer Moschusverbindungen, vorzugsweise einer oder mehrerer makrocyclischer Moschusverbindungen, umfassend den folgenden Schritt:
- Vermischen einer oder mehrerer Moschusverbindungen, vorzugsweise einer oder mehrerer makrocyclischer Moschusverbindungen, mit einer Gesamtmenge an Verbindungen (**A-1**) und (**A-2**) (Bestandteil (a)) wie in Anspruch 1 definiert, die ausreicht, die metallische Note, insbesondere die Bügeleisennote, einer, mehrerer oder sämtlicher der Moschusverbindungen zu modifizieren, vorzugsweise zu verringern oder zu unterdrücken.

## Claims

1. A fragrance composition, preferably a perfume oil, comprising
(a) component (A-1) and component (A-2) 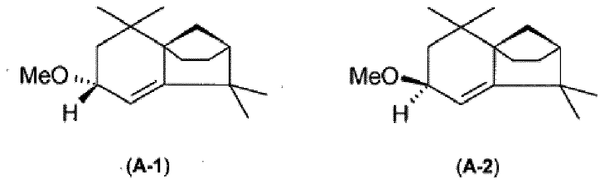 and
(b) one or more musk fragrances different from isolongifolenyl methyl ether,
whereby preferably the mass ratio of the total amount of musk fragrances forming component (b) to the total amount of component (a-1) and (a-2) (component a) is equal or above 1:2, preferably equal or above 1:1 and more preferably equal or above 3:2.

2. The fragrance composition, preferably the perfume oil of Claim 1, whereby the mass ratio of the total amount of musk fragrances forming component (b) to the total amount of the components forming component (a) is in the range of 3:2 to 20:1, preferably in the range of 3:2 to 15:1, more preferably in the range of 2:1 to 12:1.

3. The fragrance composition, preferably the perfume oil of one of the preceding claims, whereby the additional musk fragrances forming component (b) are selected from the group consisting of macrocyclic musk fragrances, nitro musk fragrances, polycyclic musk fragrances and/or alicyclic musk fragrances.

4. The fragrance composition, preferably the perfume oil of one of the preceding claims, whereby the mass ratio of the total amount of the isomer (A-1) to the total amount of the isomer (a-2) is equal or above 11:4, preferably equal or above 3:1, more preferably equal or above 7:2, more preferably in the range of 3:1 to 12:1, more preferably in the range of 7:2 to 10:1, even more preferably in the range of 15:4 to 10:1, and most preferably in the range of 4:1 to 8:1.

5. The fragrance composition, preferably the perfume oil of one of the preceding claims, whereby the additionally contain
(c) one, 2, 3, 4 or more terpene alcohols having the sum formula C10HO, in which stands for the numbers 18, 20 or 22, and whereby preferably one or more or all terpene alcohols are selected from the group consisting of linalool, eucalyptol, dihydromyrcenol, citronellol, nerol, geraniol, menthol, tetrahydrolinalool and tetrahydrogeraniol;
and/or
(d) one, 2, 3 or more additional wood-like fragrances, whereby said additional wood-like fragrance or the additional wood-like fragrances forming component (d) are different from component (a) and component (b).

6. The fragrance composition, preferably the perfume oil of Claim 5, whereby the mass ratio of the total amount of terpene alcohols (component c) to the total amount of components (A-1) and (a-2) (component a) is equal or above 1:1, preferably equal or above 2:1, more preferably equal or above 3:1, and most preferably equal or above 4:1.

7. The fragrance composition, preferably the perfume oil of one of the preceding claims, whereby, whereby component (d) encompasses or consists of one, two, three or more wood-like fragrances, selected from the group consisting of
(d) (i) 2,3,8,8-tetramethyl-1,2,3,4,5,6,7,8-octohydro-2-naphthalenylmethylketon and/or 1-[1,2,3,4,5,6,7,8,8a-octahydro-1,2,8,8-tetramethylnaphthalen-2-yl]ethanon,
and/or
(d) (ii) sandal wood fragrances, preferably selected from the group consisting of 2-methyl-4-(2,2,3-trimethyl-3-cyclopenten-1-yl)butanol, 5-(2,2,3-trimethyl-3-cyclopenten-1-yl)-3-methylpentan-2-ol, 2-ethyl-4-(2,2,3-trimethyl-3-caclopenten-1-yl)-2-buten-1-ol, 3-methyl-5-(2,2,3-trimethyl-3-cyclopenten-1-yl)-4-penten-2-ol, 3,3-dimethyl-5-(2,2,3-trimethyl-3-cyclopenten-1-yl)-4-penten-2-ol, 3-isocamphylcycloheanol and [1-methyl-2-(1,2,2-trimethyl[3.1.0]hex-3-ylmethyl)cyclopropyl]methanol.

8. The fragrance composition, preferably the perfume oil of Claim 7, whereby the mass ratio of the total amount of component (d) (i) 2,3,8,8-tetramethyl-1,2,3,4,5,6,7,8octahydro-2-naphthalenylmethylketon and 1-[1,2,3,4,6,7,8,8a-octahydro-1,2,8,8-tetramethylnaphthalen-2-yl]ethanon to the total amount of components (A-1) and (A-2) (component a) is equal or above 1:1, preferably equal or above 2:1, more preferably equal or above 3:1, most preferably equal or above 4:1.

9. The fragrance composition, preferably the perfume oil of Claim 7 or Claim 8, whereby the mass ratio of the total amount of sandal wood fragrances (component (d) (ii)) to the total amount of components (A-1) and (A-2) (component a) is equal or above 3:1, preferably equal or above 4:1.

10. The fragrance composition, preferably the perfume oil of one of the preceding claims, whereby component
(b) encompasses one or more macro-cyclic musk fragrances, preferably selected from the group consisting of macro-cyclic C₁₄-C₁₆-ketones and macro-cyclic C₁₄-C₁₆ lactones, whereby said ketone or lactone respectively preferably shows a ring size of 15 to 17 ring atoms and one, one or two oxygen atoms in the ring.

11. The fragrance composition, preferably the perfume oil of one of the preceding claims, whereby component
(b) represents or encompasses 15-pentadec-(11/12)-enolide, ethylenbrassylate, 15-pentadecanolide, 5-cycloheadecen-1-one, 7-cycloheadecen-1-one, 8-cycloheadecen-1-one, 7/8-cyclohexadecen-1-one, cyclohexadecanone, 3-methylcyclopentadecanone, 3.methylcyclopentadecenone and/or 9-cycloheptadecen-1-one.

12. A perfumed product comprising a fragrance composition, preferably a perfume oil, according to one of the preceding claims, preferably in a sensory working amount.

13. The perfumed product of Claim 12, selected from the group consisting of detergent and cleaning products, personal care products, preferably for body care, hair care, cosmetics and household products.

14. A fragrance mixture according to one of the claims 1 to 11 or a perfumed product according to claims 12 or 13, **characterised in that** the total mass of components (A-1) and (A-2) (component a) is within the range of 0.25 to 10 % b.w., preferably within the range of 0.75 to 7.5 % b.w., more preferably within the range of 1 to 6 % b.w. - each calculated on the total mass of all fragrances present in the fragrance mixture or the perfumed product.

15. Process for the modification, preferably for the reduction or suppression of metallic notes of one or more musk components, preferably one or more macro-cyclic musk components, encompassing the following steps:
- blending one or more musk components, preferably one or more macro-cyclic musk components, with a total amount of components (a-1) and (A-2) (component a) as defined in Claim 1, sufficient to modify, preferably to reduce or supress the metallic note, preferably the ironing note of one, more or even all musk components.

## Revendications

1. Composition de parfumes, de préférence d'huiles de parfume, comprenant
(a) le composé **(A-1)** et le composé (**A-2**) 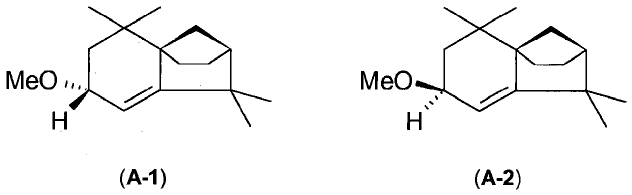 et
(b) un ou plusieurs autres parfums de musc qui ne sont pas des éthers isolongifolényl-méthyliques
de préférence le rapport massique de la quantité totale de parfums de musc du composant (b) à la quantité totale des composés (**A-1**) et (**A-2**) (composant (a)) étant supérieur ou égal à 1 : 2, de préférence supérieur ou égal à 1 : 1, encore mieux supérieur ou égal à 3 : 2.

2. Composition de parfum, de préférence huile parfumée, selon la revendication 1, dans laquelle le rapport massique de la quantité totale de parfums de musc du composant (b) à la quantité totale des composés du composant (a) se situe dans la plage de 3 : 2 à 20 : 1, de préférence dans la plage de 3 : 2 à 15 : 1, de façon particulièrement préférée dans la plage de 2 : 1 à 12 : 1.

3. Composition de parfum, de préférence huile parfumée, selon l'une quelconque des revendications précédentes, dans laquelle le ou les autres parfums de musc du composant (b) sont choisis dans le groupe des parfums de musc macrocycliques, des parfums de musc nitrés, des parfums de musc polycycliques et/ou des parfums de musc alicycliques.

4. Composition de parfum, de préférence huile parfumée, selon l'une quelconque des revendications précédentes, dans laquelle le rapport massique de la quantité totale de l'isomère **(A-1)** à la quantité totale de l'isomère (**A-2**) est supérieur ou égal à 11 : 4, de préférence supérieur ou égal à 3 : 1, encore mieux supérieur ou égal à 7 : 2, et de préférence se situe dans la plage de 3 : 1 à 12 : 1, encore mieux dans la plage de 7 : 2 à 10 : 1, de façon particulièrement préférée dans la plage de 15 : 4 à 10 : 1, et de façon tout particulièrement préférée dans la plage de 4 : 1 à 8 : 1.

5. Composition de parfum, de préférence huile parfumée, selon l'une quelconque des revendications précédentes, contenant en outre
(c) un, 2, 3, 4, 5 ou plus de 5 alcools terpéniques ayant la formule brute C₁₀HₓO, x représentant le nombre 18, 20 ou 22, et de préférence un, plusieurs ou la totalité des alcools terpéniques étant choisi(s) dans le groupe constitué par le linalol, l'eucalyptol, le dihydromyrcénol, le citronellol, le nérol, le géraniol, le menthol, le tétrahydrolinalol et le tétrahydrogéraniol,
et/ou
(d) un, 2, 3 ou plus de 3 autres parfums boisés, l'autre parfum boisé ou les autres parfums boisés du composant (d) étant différent(s) du composant (a) et du composant (b).

6. Composition de parfum, de préférence huile parfumée, selon la revendication 5, dans laquelle le rapport massique de la quantité totale d'alcools terpéniques (composant (c)) à la quantité totale des composés **(A-1)** et (**A-2**) composant (a)) est supérieur ou égal à 1 : 1, de préférence supérieur ou égal à 2 : 1, encore mieux supérieur ou égal à 3 : 1, de façon particulièrement préférée supérieur ou égal à 4 : 1.

7. Composition de parfum, de préférence huile parfumée, selon l'une quelconque des revendications précédentes, dans laquelle le composant (d) comprend ou consiste en un, deux, trois ou plus de trois parfums boisés, choisis dans le groupe constitué par
(d)(i) la 2,3,8,8-tétraméthyl-1,2,3,4,5,6,7,8-octahydro-2-naphtalénylméthylcétone et/ou la 1-[1,2,3,4,6,7,8,8a-octahydro-1,2,8,8-tétraméthylnaphtalén-2-yl]éthanone
et/ou
(d)(ii) des parfums de bois de bois de santal, de préférence choisis dans le groupe constitué par le 2-méthyl-4-(2,2,3-triméthyl-3-cyclopentén-1-yl)-butanol, le 5-(2,2,3-triméthyl-3-cyclopentén-1-yl)-3-méthylpentan-2-ol, le 2-éthyl-4-(2,2,3-triméthyl-3-cyclopentén-1-yl)-2-butén-1-ol, le 3-méthyl-5-(2,2,3-triméthyl-3-cyclopentén-1-yl)-4-pentén-2-ol, le 3,3-diméthyl-5-(2,2,3-triméthyl-3-cyclopentén-1-yl)-4-pentén-2-ol, le 3-isocamphylcyclohexanol et le [1-méthyl-2-(1,2,2-triméthyl[3.1.0]hex-3-ylméthyl)cyclopropyl]méthanol.

8. Composition de parfum, de préférence huile parfumée, selon la revendication 7, dans laquelle le rapport massique de la quantité totale du composant (d)(i) 2,3,8,8-tétraméthyl-1,2,3,4,5,6,7,8-octahydro-2-naphtalénylméthylcétone et 1-[1,2,3,4,6,7,8,8a-octahydro-1,2,8,8-tétraméthylnaphtalén-2-yl]éthanone à la quantité totale des composés (**A-1**) et (**A-2**) (composant (a)) est supérieur ou égal à 1 : 1, de préférence supérieur ou égal à 2 : 1, encore mieux supérieur ou égal à 3 : 1, de façon particulièrement préférée supérieur ou égal à 4 : 1.

9. Composition de parfum, de préférence huile parfumée, selon la revendication 7 ou 8, dans laquelle le rapport massique de la quantité totale de parfums de bois de santal (composant (d)(ii) à la quantité totale des composés (**A-1**) et (**A-2**) (composant (a)) est supérieur ou égal à 1 : 1, de préférence supérieur ou égal à 2 : 1, encore mieux supérieur ou égal à 3 : 1, de façon particulièrement préférée supérieur ou égal à 4 : 1.

10. Composition de parfum, de préférence huile parfumée, selon l'une quelconque des revendications précédentes, dans laquelle le composant
(b) contient un ou plusieurs parfums de musc macrocycliques, de préférence choisis dans le groupe constitué par des cétones en C₁₄-C₁₈ macrocycliques et des lactones en C₁₄-C₁₈ macrocycliques, la cétone ou lactone ayant de préférence une taille de cycle de 15 à 17 atomes formant le cycle et ne comportant aucun atome d'oxygène ou comportant un ou deux atomes d'oxygène dans le cycle.

11. Composition de parfum, de préférence huile parfumée, selon l'une quelconque des revendications précédentes, dans laquelle le composant
(b) est ou contient le 15-pentadéc-(11/12)-énolide, le brassylate d'éthylène, le 15-pentadécanolide, la 5-cyclohexadécén-1-one, la 7-cyclohexadécén-1-one, la 8-cyclohexadécén-1-one, la 7/8-cyclohexadécén-1-one, la cyclohexadécanone, la 3-méthylcyclopentadécanone, la 3-méthylcyclopentadécénone et/ou la 9-cycloheptadécén-1-one.

12. Produit parfumé contenant une composition de parfum, de préférence une huile parfumée, selon l'une quelconque des revendications précédentes, de préférence en une quantité à efficacité sensorielle.

13. Produit parfumé selon la revendication 12, choisi dans le groupe constitué par des produits de lavage et de nettoyage et des produits de soin et d'hygiène, en particulier dans le domaine du soin du corps, du soin des cheveux, de la cosmétique et le secteur ménager.

14. Composition de parfum selon l'une quelconque des revendications 1 à 11 ou produit parfumé selon la revendication 12 ou 13, **caractérisés en ce que** la masse totale des composés (**A-1**) et (**A-2**) (composant (a)) se situe dans la plage de 0,25 à 10 % en poids, de préférence dans la plage de 0,5 à 8 % en poids, de façon particulièrement préférée dans la plage de 0,75 à 7,5 % en poids, de façon plus particulièrement préférée dans la plage de 1 à 6 % en poids, chaque fois par rapport à la masse totale de tous les parfums contenus dans la composition de parfum ou le produit parfumé.

15. Procédé pour la modification, de préférence pour la diminution ou le masquage d'une note métallique d'un ou de plusieurs composés de musc, de préférence d'un ou de plusieurs composés de musc macrocycliques, comprenant l'étape suivante :
- mélange d'un ou de plusieurs composés de musc, de préférence d'un ou de plusieurs composés de musc macrocycliques, avec une quantité totale de composés (**A-1**) et (**A-2**) (composant (a)) tels que définis dans la revendication 1, qui est suffisante pour modifier, de préférence pour diminuer ou masquer, la note métallique, en particulier la note de roussi, d'un, de plusieurs ou de la totalité des composés de musc.
